# EUROPEAN PATENT APPLICATION

(11) **EP 4 821 955 A1**
(43) Date of publication of application: **14.10.2026**
(21) Application number: 25842578.4
(22) Date of filing: 25.02.2025
(51) Int. Cl.: C08B 37/08, A61K 31/715, A61P 7/02

(54) **MARINE SODIUM POLYSACCHARIDE, METHOD FOR PREPARING SAME, USE THEREOF, AND ANTICOAGULANT AND/OR ANTITHROMBOTIC MEDICAMENT TARGETING INTRINSIC COAGULATION PATHWAY**

(30) Priority: 21.02.2025 CN 202510197314
(71) Applicant: Harbin Hongdoushan Bio-pharm Co., Ltd., Harbin, Heilongjiang 150600 (CN)
(72) Inventor: YANG, Lei, Harbin, Heilongjiang 150600 (CN); HAN, Li, Harbin, Heilongjiang 150600 (CN); WANG, Yicheng, Harbin, Heilongjiang 150600 (CN); YANG, Baofeng, Harbin, Heilongjiang 150600 (CN); HUANG, Xueshi, Harbin, Heilongjiang 150600 (CN); WANG, Zhiguo, Harbin, Heilongjiang 150600 (CN); WANG, Yiming, Harbin, Heilongjiang 150600 (CN); QI, Guifang, Harbin, Heilongjiang 150600 (CN)
(74) Representative: LEITZINGER OY
(86) International application number: PCT/CN2025/078954
(87) International publication number: WO 2026/174607

(57) **Abstract**

A Haina polysaccharide (HPS) and a preparation method and use thereof, and an anticoagulant drug and/or an antithrombotic drug targeting an intrinsic coagulation pathway are provided, relating to a technical field of pharmaceuticals. The HPS is a fucosylated chondroitin sulfate polysaccharide; the HPS has a weight-average molecular weight of 90,000 to 130,000, where a molar ratio of glucuronic acid, N-acetyl-D-galactosamine, and fucose is in a range of 1 : 0.8-1.2 : 0.5-0.8; and a sulfate group in the HPS has a mass percentage of 25% to 40%. The HPS can target a terminal rate-limiting enzyme in the intrinsic coagulation pathway (iFXase), with insignificant effects on coagulation factors in other coagulation pathways, thereby achieving anticoagulation without bleeding. The HPS can be widely used in the treatment of both the acute and recovery phases of ischemic stroke, and solves the safety issues associated with the use of anticoagulant and antithrombotic drugs in clinical patients with ischemic stroke. Therefore, the HPS pioneers a new field in the development of anticoagulant drugs targeting a rate-limiting enzyme of the intrinsic coagulation pathway.

## Description

### TECHNICAL FIELD

The present disclosure relates to the technical field of pharmaceuticals, and in particular to a Haina polysaccharide (HPS) and a preparation method and use thereof, and an anticoagulant drug and/or an antithrombotic drug targeting an intrinsic coagulation pathway.

### BACKGROUND

Anticoagulant and/or antithrombotic drugs are a class of drugs that inhibit blood coagulation by interfering with the body's coagulation process, thereby preventing thrombus formation or halting a further progression of existing thrombi. The timely administration of anticoagulant and/or antithrombotic drugs during the acute and subacute phases of ischemic stroke is crucial for alleviating symptoms. The coagulation pathways in human body are divided into an intrinsic coagulation pathway, an extrinsic coagulation pathway, and a common pathway. The intrinsic coagulation pathway leads to pathological thrombosis, while the extrinsic coagulation pathway is associated with inhibiting vascular wall damage, and the common pathway influences both. Intrinsic coagulation factor Xase (Intrinsic Tenase, FIXa-FVIIIa-PL-Ca²⁺ complex, iFXase) is a terminal rate-limiting enzyme of the intrinsic coagulation pathway. The intrinsic coagulation pathway is closely related to pathological thrombus formation and is not essential for normal hemostatic function. Currently, clinically used anticoagulant and/or antithrombotic drugs directly target coagulation factor IIa and/or coagulation factor Xa in the common pathway. Due to bleeding side effects, current international and domestic guidelines do not recommend the early use of direct IIa inhibitors or Xa factor inhibitors. Anticoagulant and/or antithrombotic drugs targeting the intrinsic coagulation pathway have been a major focus of global research, yet no related drugs have been marketed to date.

The body wall of sea cucumbers is rich in polysaccharides, primarily including two major categories: fucoidan and fucosylated chondroitin sulfate. Among these, sea cucumber-derived fucosylated chondroitin sulfate exhibits a wide range of biological activities due to its unique structure, such as lipid-lowering, antitumor, antiviral, and anti-inflammatory effects. Notably, this component exhibits excellent anticoagulant and antithrombotic activities. However, the natural fucosylated chondroitin sulfate extracted from the sea cucumber body wall typically carries a significant risk of bleeding side effects, that is to say, it cannot specifically target the intrinsic coagulation pathway. In related techniques, the extracted natural polysaccharides are degraded to reduce the molecular weight of sea cucumber-derived fucosylated chondroitin sulfate, thereby minimizing bleeding side effects. However, this process simultaneously leads to a reduction in anticoagulant efficacy.

In summary, there is an urgent need to provide an anticoagulant drug and/or an antithrombotic drug that can target the intrinsic coagulation pathway, with good anticoagulant effects and minimal bleeding side effects.

### SUMMARY

In view of this, an object of the present disclosure is to provide a Haina polysaccharide (HPS) and a preparation method and use thereof, and an anticoagulant drug and/or an antithrombotic drug targeting an intrinsic coagulation pathway. In the present disclosure, the HPS is a natural fucosylated chondroitin sulfate polysaccharide directly extracted from sea cucumber body wall, which can target a terminal rate-limiting enzyme iFXase in an intrinsic coagulation pathway. The HPS provides excellent anticoagulant effects with minimal bleeding side effects, thereby achieving effective anticoagulation without causing bleeding.

To achieve the above objects, the present disclosure provides the following technical solutions:

The present disclosure provides an HPS, where the HPS is a fucosylated chondroitin sulfate polysaccharide; the HPS has a weight-average molecular weight of 90,000 to 130,000, and is composed of monosaccharides including glucuronic acid, N-acetyl-D-galactosamine, and fucose, where a molar ratio of the glucuronic acid, the N-acetyl-D-galactosamine, and the fucose is in a range of 1 : 0.8-1.2 : 0.5-0.8; and a sulfate group in the HPS has a mass percentage of 25% to 40%.

In some embodiments, the HPS has a structural unit shown in formula I: 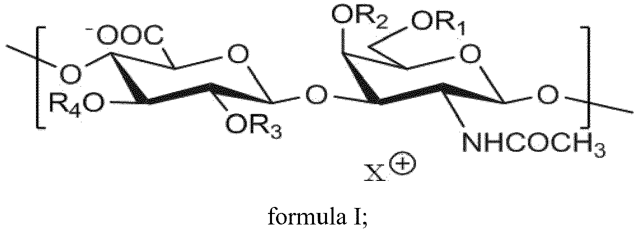
where in the formula I, R₁ is selected from the group consisting of H, SO₃⁻, and fucosyl; R₂ is selected from the group consisting of H and SO₃⁻; R₃ is selected from the group consisting of H and SO₃⁻; R₄ is selected from the group consisting of H and fucosyl; at least one of the R₁ and the R₄ is fucosyl; and X^{⊕} represents a cation;
the fucosyl for the R₁ and the R₄ has a structure shown in formula I-1: 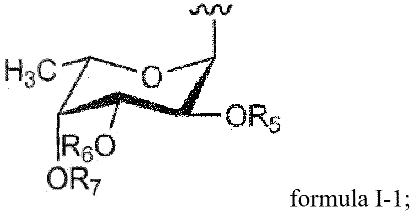
where in the formula I-1, R₅ is selected from the group consisting of H and SO₃⁻; R₆ is selected from the group consisting of H and SO₃⁻; and R₇ is selected from the group consisting of H and SO₃⁻.

In some embodiments, in a hydrogen nuclear magnetic resonance (¹H NMR) spectrum of the HPS, a signal peak for anomeric protons of a fucose moiety is present at a relative chemical shift of 5.70 ppm to 4.90 ppm; a signal peak for anomeric protons of a glucuronic acid moiety is present at a relative chemical shift of 4.80 ppm to 4.45 ppm; a signal peak for anomeric protons of an N-acetyl-D-galactosamine moiety is present at a relative chemical shift of 4.90 ppm to 4.50 ppm; a signal peak for acetyl protons of the N-acetyl-D-galactosamine moiety is present at a relative chemical shift of 2.40 ppm to 1.80 ppm; and a signal peak for methyl protons of the fucose moiety is present at a relative chemical shift of 1.60 ppm to 1.10 ppm;
in a carbon nuclear magnetic resonance (¹³C NMR) spectrum of the HPS, a signal peak for anomeric carbons of the glucuronic acid moiety is present at a relative chemical shift of 107.0 ppm to 105.0 ppm; a signal peak for anomeric carbons of the N-acetyl-D-galactosamine moiety is present at a relative chemical shift of 103.0 ppm to 101.0 ppm; a signal peak for anomeric carbons of the fucose moiety is present at relative chemical shifts of 104.0 ppm to 103.0 ppm and 101.0 ppm to 98.0 ppm; a signal peak for 2-position carbon of the N-acetyl-D-galactosamine moiety is present at a relative chemical shift of 56.0 ppm to 51.0 ppm; a signal peak for acetyl carbons of the N-acetyl-D-galactosamine moiety is present at a relative chemical shift of 27.0 ppm to 24.0 ppm; and a signal peak for methyl carbon of the fucose moiety is present at a relative chemical shift of 20.0 ppm to 16.0 ppm; and
during measurement of the ¹H NMR spectrum and the ¹³C NMR spectrum of the HPS, deuterium oxide is used as a solvent, and sodium 3-(trimethylsilyl)propionate-2,2,3,3-d4 is used as an internal standard.

In some embodiments, the cation is selected from the group consisting of hydrogen ion, an alkali metal ion, an alkaline earth metal ion, an ammonium ion, and an iron ion.

In some embodiments, the cation is sodium ion; and the sodium ion in the HPS has a mass percentage of 5% to 15%.

In some embodiments, the HPS has a polydispersity index less than or equal to 2.

The present disclosure further provides a preparation method of the HPS, including the following steps:
(1) subjecting a body wall powder of a sea cucumber to enzymolysis and acid hydrolysis in sequence to obtain an acid hydrolysate supernatant, where an enzyme used in the enzymolysis is a protease;
(2) adjusting a pH value of the acid hydrolysate supernatant to 6.5 to 7.5, conducting alcohol precipitation, and subjecting an obtained alcohol precipitation solid to washing, centrifugation, and freeze-drying in sequence to obtain a crude polysaccharide product; and
(3) subjecting the crude polysaccharide product to column chromatography purification, collecting a fraction having a weight-average molecular weight of 90,000 to 130,000, and subjecting the fraction to alcohol precipitation to obtain the HPS.

The present disclosure further provides use of the HPS as described in above technical solutions or the HPS prepared by the preparation method as described in above technical solutions in manufacture of an anticoagulant drug and/or an antithrombotic drug or an inhibitor targeting intrinsic tenase (iFXase).

The present disclosure further provides an inhibitor targeting iFXase, including the HPS as described in above technical solutions or the HPS prepared by the preparation method as described in above technical solutions.

The present disclosure further provides an anticoagulant drug and/or an antithrombotic drug targeting an intrinsic coagulation pathway, including: an active ingredient and a pharmaceutically acceptable auxiliary material, where the active ingredient is the HPS as described in above technical solutions or the HPS prepared by the preparation method as described in above technical solutions.

The present disclosure provides an HPS, where the HPS is a fucosylated chondroitin sulfate polysaccharide; the HPS has a weight-average molecular weight of 90,000 to 130,000, and is composed of monosaccharides including glucuronic acid, N-acetyl-D-galactosamine, and fucose, where a molar ratio of the glucuronic acid, the N-acetyl-D-galactosamine, and the fucose is in a range of 1 : 0.8-1.2 : 0.5-0.8; and a sulfate group in the HPS has a mass percentage of 25% to 40%. The HPS can target a terminal rate-limiting enzyme (iFXase) in the intrinsic coagulation pathway, with insignificant effects on coagulation factors in other coagulation pathways at therapeutic doses, thereby achieving the effect of anticoagulation without bleeding. The HPS can be widely used in the treatment of both the acute and recovery phases of ischemic stroke, reduces the risk of bleeding accompanying with anticoagulation in the treatment of thrombotic diseases, and solves the safety issues associated with the use of anticoagulant and antithrombotic drugs in clinical patients with ischemic stroke. Therefore, the HPS pioneers a new field in the development of anticoagulant drugs targeting the rate-limiting enzyme of the intrinsic coagulation pathway.

In the present disclosure, evaluation results of the anticoagulant activity of HPS in mice *in vivo* indicate that HPS can significantly prolong the activated partial thromboplastin time (APTT) in mice and rats, without affecting the prothrombin time (PT) and thrombin time (TT). These results suggest that the HPS targets the intrinsic coagulation pathway and does not affect the extrinsic coagulation pathway.

In the present disclosure, referring to the pharmacopoeia general chapter 1208 "heparin biological assay method", tests are conducted for the anti-Factor IIa activity, anti-Factor Xa activity, AT-III-IIa activity, and AT-III-Xa activity of HPS. The results show that at an experimental dose of 500 µg/mL, HPS displays no activity against Factor IIa, Factor Xa, or AT-III-IIa, and only shows weak effect against AT-III-Xa (with an inhibition rate of 36.7%). Using the enzyme-linked immunosorbent assay (ELISA), activity testing was conducted on the rate-limiting enzyme of the intrinsic coagulation pathway, "intrinsic factor coagulation factor Xase (iFXase)". The results indicate that the test drug can significantly inhibit the intrinsic coagulation pathway rate-limiting enzyme iFXase, with an IC₅₀ value of 207.5 ng/mL. The above experiments further clarify, mechanistically, that the HPS can selectively inhibit the activity of the rate-limiting enzyme iFXase in the intrinsic coagulation pathway, targeting the intrinsic coagulation pathway.

### BRIEF DESCRIPTION OF THE DRAWINGS

FIG. 1 shows the linear regression analysis results for sodium ions in Example 4;
FIG. 2 shows the linear regression analysis results for sulfate groups in Example 5;
FIG. 3 shows the hydrogen nuclear magnetic resonance (¹H NMR) spectrum of the HPS in Example 6;
FIG. 4 shows the carbon nuclear magnetic resonance (¹³C NMR) spectrum of the HPS in Example 6;
FIG. 5 shows the heteronuclear singular quantum correlation (HSQC) test spectrum of the HPS in Example 6;
FIG. 6 shows the effect of the HPS on coagulation function in mice in Example 7, where panel A shows the TT test results, panel B shows the APTT test results, and panel C shows the PT test results;
FIG. 7 shows the effect of the HPS on venous thrombosis in mice in Example 8, where panel A shows images of thrombus length in mouse tails, and panel B shows the percentage of thrombus length relative to total tail length; and
FIG. 8 shows the test mechanism of the FVIII activity assay kit used in Example 10.

### DETAILED DESCRIPTION OF THE EMBODIMENTS

In the present disclosure, HPS specifically refers to a natural polysaccharide extracted from body wall(s) of a sea cucumber.

The present disclosure provides an HPS, where the HPS is a fucosylated chondroitin sulfate polysaccharide; the HPS has a weight-average molecular weight of 90,000 to 130,000, and is composed of monosaccharides including glucuronic acid, N-acetyl-D-galactosamine, and fucose, where a molar ratio of the glucuronic acid, the N-acetyl-D-galactosamine, and the fucose is in a range of 1 : 0.8-1.2 : 0.5-0.8; and a sulfate group in the HPS has a mass percentage of 25% to 40%.

In the present disclosure, the HPS is a fucosylated chondroitin sulfate polysaccharide, whose backbone is a copolymer of disaccharide units composed of N-acetyl-D-galactosamine and glucuronic acid, and the disaccharide units are linked with fucose branch chains; the HPS has a weight-average molecular weight of 90,000 to 130,000, preferably 90,000 to 120,000; in some embodiments, the HPS has a polydispersity index less than or equal to 2, preferably less than or equal to 1.32, specifically 1.27 to 1.32.

In the present disclosure, a molar ratio of the glucuronic acid, the N-acetyl-D-galactosamine, and the fucose in the HPS is in a range of 1 : 0.8-1.2 : 0.5-0.8, preferably 1 : 0.9-1.1 : 0.6-0.7.

In the present disclosure, a sulfate group in the HPS has a mass percentage of 25% to 40%, preferably 26% to 35% (determined by ion chromatography) or 32% to 40% (determined by colorimetric method).

In some embodiments of the present disclosure, the HPS has a structural unit shown in formula I: 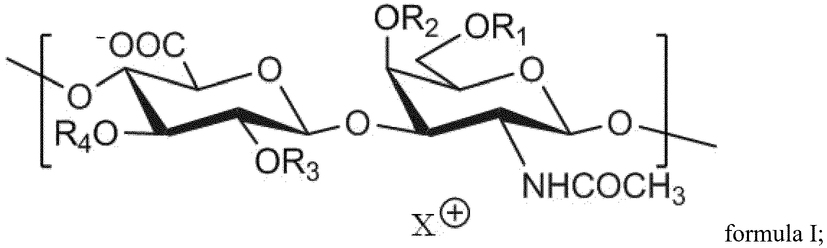
where in the formula I, R₁ is selected from the group consisting of H, SO₃⁻, and fucosyl; R₂ is selected from the group consisting of H and SO₃⁻; R₃ is selected from the group consisting of H and SO₃⁻; R₄ is selected from the group consisting of H and fucosyl; at least one of R₁ and R₄ is fucosyl; and X^{⊕} represents a cation;
the fucosyl for the R₁ and the R₄ has a structure shown in formula I-1: 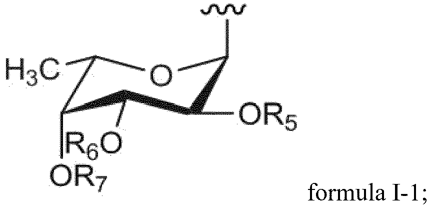
where in the formula I-1, R₅ is selected from the group consisting of H and SO₃⁻; R₆ is selected from the group consisting of H and SO₃⁻; and R₇ is selected from the group consisting of H and SO₃⁻.

In some embodiments of the present disclosure, the cation in formula I is hydrogen ion, an alkali metal ion, an alkaline earth metal ion, an ammonium ion, or an iron ion; the alkali metal ion is preferably sodium ion or potassium ion; the alkaline earth metal ion is preferably a calcium ion; in specific examples, the cation in formula I is preferably sodium ion; when the cation is sodium ion, the sodium ion in the HPS has a mass percentage of preferably 5% to 15%, more preferably 8% to 11%.

In the present disclosure, in a hydrogen nuclear magnetic resonance (¹H NMR) spectrum of the HPS, a signal peak for anomeric protons of a fucose moiety is present at a relative chemical shift of 5.70 ppm to 4.90 ppm; a signal peak for anomeric protons of a glucuronic acid moiety is present at a relative chemical shift of 4.80 ppm to 4.45 ppm; a signal peak for anomeric protons of an N-acetyl-D-galactosamine moiety is present at a relative chemical shift of 4.90 ppm to 4.50 ppm; a signal peak for acetyl protons of the N-acetyl-D-galactosamine moiety is present at a relative chemical shift of 2.40 ppm to 1.80 ppm; and a signal peak for methyl protons of the fucose moiety is present at a relative chemical shift of 1.60 ppm to 1.10 ppm;
in a carbon nuclear magnetic resonance (¹³C NMR) spectrum of the HPS, a signal peak for anomeric carbons of the glucuronic acid moiety is present at a relative chemical shift of 107.0 ppm to 105.0 ppm; a signal peak for anomeric carbons of the N-acetyl-D-galactosamine moiety is present at a relative chemical shift of 103.0 ppm to 101.0 ppm; a signal peak for anomeric carbons of the fucose moiety is present at relative chemical shifts of 104.0 ppm to 103.0 ppm and 101.0 ppm to 98.0 ppm; a signal peak for 2-position carbon of the N-acetyl-D-galactosamine moiety is present at a relative chemical shift of 56.0 ppm to 51.0 ppm; a signal peak for acetyl carbons of the N-acetyl-D-galactosamine moiety is present at a relative chemical shift of 27.0 ppm to 24.0 ppm; and a signal peak for methyl carbon of the fucose moiety is present at a relative chemical shift of 20.0 ppm to 16.0 ppm.

In some embodiments of the present disclosure, during measurement of the ¹H NMR spectrum and the ¹³C NMR spectrum of the HPS, deuterium oxide is used as a solvent, and sodium 3-(trimethylsilyl)propionate-2,2,3,3-d4 is used as an internal standard. A signal peak for methyl protons of the sodium 3-(trimethylsilyl)propionate-2,2,3,3-d4 is defined as 0.00 ppm.

In the present disclosure, the HPS is extracted from body wall(s) of a sea cucumber; the sea cucumber includes, but is not limited to, one or more of *Holothuria leucospilota, Holothuria scabra, Stichopus japonicus, Holothuria atra, and Holothuria nobilis,* preferably *Holothuria leucospilota.*

The present disclosure further provides a preparation method of the HPS, including the following steps:
(1) subjecting a body wall powder of a sea cucumber to enzymolysis and acid hydrolysis in sequence to obtain an acid hydrolysate supernatant, where an enzyme used in the enzymolysis is a protease;
(2) adjusting a pH value of the acid hydrolysate supernatant to 6.5 to 7.5, conducting alcohol precipitation, and subjecting an obtained alcohol precipitation solid to washing, centrifugation, and freeze-drying in sequence to obtain a crude polysaccharide product; and
(3) subjecting the crude polysaccharide product to column chromatography purification, collecting a fraction having a weight-average molecular weight of 90,000 to 130,000, and subjecting the fraction to alcohol precipitation to obtain the HPS.

In the present disclosure, the body wall powder of the sea cucumber is subjected to enzymolysis and acid hydrolysis in sequence, thereby obtaining an acid hydrolysate supernatant. In some embodiments, the body wall powder of the sea cucumber is prepared by a process including: soaking the body wall of the sea cucumber in acetone, followed by drying and grinding to obtain the body wall powder of the sea cucumber, where the soaking is conducted at a temperature of preferably 3°C to 5°C, more preferably 4°C for preferably 12 h to 48 h, more preferably 24 h. There are no specific requirements for the drying and grinding, and conditions well-known to those skilled in the art may be used. The species of sea cucumber are not reiterated here.

In some embodiments of the present disclosure, the protease is papain; in some embodiments, the body wall powder of the sea cucumber is suspended in a sodium acetate buffer containing the papain for enzymolysis; preferably, a weight ratio of the body wall powder of the sea cucumber to the papain is in a range of (5-15):1, more preferably 10:1; the sodium acetate buffer preferably has a concentration of 0.1 mol/L, and a pH value of preferably 6; the enzymolysis is preferably conducted at a temperature of 50°C to 70°C, more preferably 65°C for preferably 12 h to 48 h, more preferably 24 h. After the enzymolysis is completed, a resulting mixture is centrifuged (recorded as the first centrifugation) to obtain a supernatant, and the supernatant is subjected to the acid hydrolysis; the first centrifugation is preferably conducted at a temperature of 3°C to 5°C, more preferably 4°C with a rotational speed of preferably 3,000 rpm to 5,000 rpm, more preferably 4,000 rpm for preferably 20 min to 40 min, more preferably 30 min.

In some embodiments of the present disclosure, an acid used for the acid hydrolysis is hydrochloric acid, and the hydrochloric acid preferably has a concentration of 6 mol/L; in some embodiments, the acid hydrolysis is conducted at a pH value of 2 to 3 (specifically 2.5) for preferably 1 h to 3 h, more preferably 2 h, at preferably room temperature, preferably under stirring. In some embodiments, after the acid hydrolysis is completed, a resulting mixture is centrifuged (recorded as the second centrifugation) to obtain the acid hydrolysate supernatant; the second centrifugation is preferably conducted at a temperature of 3°C to 5°C, preferably 4°C with a rotational speed of preferably 3,000 rpm to 5,000 rpm, more preferably 4,000 rpm for preferably 20 min to 40 min, more preferably 30 min.

In the present disclosure, after obtaining the acid hydrolysate supernatant, a pH value of the acid hydrolysate supernatant is adjusted to 6.5 to 7.5, and alcohol precipitation is conducted to obtain an alcohol precipitation solid. In some embodiments, a reagent used to adjust the pH value of the acid hydrolysate supernatant is an aqueous sodium hydroxide solution, and the aqueous sodium hydroxide solution preferably has a mass concentration of 30% to 50%, more preferably 40%; an alcohol used for the alcohol precipitation is preferably ethanol, and the ethanol preferably has a volume fraction of 90% to 95%, more preferably 95%; the alcohol precipitation is preferably conducted at a temperature of -5°C to -15°C, preferably -10°C, preferably 2 to 3 times, with a duration of each alcohol precipitation preferably being 20 h to 30 h, more preferably 24 h. Specifically, in some embodiments, the ethanol is first added to the acid hydrolysate supernatant and the first alcohol precipitation is conducted, a precipitate is collected after centrifugation (recorded as the third centrifugation), the precipitate is dissolved in distilled water, and then the ethanol is added thereto for a second alcohol precipitation, and a precipitate is collected after centrifugation (recorded as the fourth centrifugation) to obtain the alcohol precipitation solid. In some embodiments, a volume ratio of the ethanol used in the first alcohol precipitation to the acid hydrolysate supernatant is in a range of 1 : 0.5-1.5, preferably 1:1; preferably, a volume ratio of the ethanol used in the second alcohol precipitation and the distilled water used to dissolve the precipitate is in a range of (1-3):1, preferably 2:1; preferably, the third centrifugation and the fourth centrifugation each are conducted at a temperature of 3°C to 5°C, more preferably 4°C with a rotational speed of preferably 3,000 rpm to 5,000 rpm, more preferably 4,000 rpm for preferably 20 min to 40 min, more preferably 30 min.

In the present disclosure, after obtaining the alcohol precipitation solid, the alcohol precipitation solid is sequentially subjected to washing, centrifugation (recorded as the fifth centrifugation), and freeze-drying to obtain a crude polysaccharide product; a washing agent used for the washing is preferably ethanol, and the ethanol preferably has a volume fraction of 95%; preferably, the fifth centrifugation is conducted at a temperature of 3°C to 5°C, more preferably 4°C with a rotational speed of preferably 3,000 rpm to 5,000 rpm, more preferably 4,000 rpm for preferably 20 min to 40 min, more preferably 30 min; preferably, the freeze-drying specifically involves dissolving a washed solid in distilled water, and then conducting the freeze-drying.

In the present disclosure, after obtaining the crude polysaccharide product, the crude polysaccharide product is subjected to column chromatography purification, a fraction having a weight-average molecular weight of 90,000 to 130,000 is collected, and the fraction is subjected to alcohol precipitation, thereby obtaining the HPS. In some embodiments, A chromatography column used for the column chromatography purification is a diethylaminoethylcellulose (DEAE) cellulose column, and a specification of the DEAE cellulose column is preferably 10 cm × 4 cm; preferably, the DEAE cellulose column is pre-equilibrated with an acetic acid-sodium acetate buffer, and then the crude polysaccharide product is loaded onto a pre-equilibrated DEAE cellulose column; an elution process in the column chromatography purification includes a first stage and a second stage, where an eluent used in both stages is an acetic acid-sodium acetate buffer containing sodium chloride; a concentration of sodium chloride in the eluent used in the first stage is in a range of 0.4 mol/L to 0.6 mol/L, preferably 0.5 mol/L, and a concentration of sodium chloride in the eluent used in the second stage is in a range of 0.8 mol/L to 1.2 mol/L, preferably 1 mol/L. In specific examples, each fraction is collected in 500 mL during the elution process, and an HPLC method is conducted on each fraction for molecular weight and molecular weight distribution, and a chromatographic peak fraction with a weight-average molecular weight of 90,000 to 130,000 is collected.

In some embodiments of the present disclosure, the alcohol precipitation is conducted once on the fraction, an alcohol used for the alcohol precipitation is preferably ethanol, a volume fraction of the ethanol is preferably 95%, preferably, a volume ratio of the ethanol to the fraction are is in a range of (0.5-1.5):1, more preferably 1:1; preferably, the alcohol precipitation is conducted at a temperature of -5°C to -15°C, more preferably -10°C for preferably 20 h to 30 h, more preferably 24 h. After the alcohol precipitation is completed, centrifugation is conducted (recorded as the sixth centrifugation) to collect a precipitate; preferably, the sixth centrifugation is conducted at a temperature of 3°C to 5°C, more preferably 4°C with a rotational speed of preferably 3,000 rpm to 5,000 rpm, more preferably 4,000 rpm for preferably 20 min to 40 min, more preferably 30 min.

In the present disclosure, after the sixth centrifugation is completed, an obtained precipitate is preferably sequentially subjected to concentration and freeze-drying; preferably, the concentration is conducted by using ultrafiltration membrane, and a molecular weight cut-off of the ultrafiltration membrane is preferably 10,000; the concentration is conducted preferably 4 times. In some embodiments, specifically, the precipitate obtained from the sixth centrifugation is dissolved in distilled water, concentrated to one-half the original volume using the ultrafiltration membrane, distilled water is then supplemented to the original volume, and a resulting mixture is then concentrated to one-half the original again using the ultrafiltration membrane, the concentration process are performed similarly 4 times to obtain a concentrate. After the concentrate is obtained, the concentrate is freeze-dried to obtain the pure HPS. There are no specific requirements for the conditions of the freeze-drying, and those well-known to those skilled in the art may be used.

In the present disclosure, the cation in the HPS extracted according to the above method is sodium ion; in specific examples, the HPS can be further subjected to cation exchange to obtain an HPS with other cations; there are no specific requirements for the method of cation exchange, and methods well-known to those skilled in the art may be used.

The present disclosure further provides use of the HPS or the HPS prepared by the preparation method in manufacture of an anticoagulant drug and/or an antithrombotic drug, or an inhibitor targeting intrinsic tenase (iFXase). In the present disclosure, there are no specific requirements for the use, and methods well-known to those skilled in the art may be used.

The present disclosure further provides an inhibitor targeting iFXase, where an active ingredient of the inhibitor targeting iFXase includes the HPS as described in above technical solutions or the HPS prepared by the preparation method as described above. In the present disclosure, there are no specific requirements for other components in the inhibitor targeting iFXase, and those well-known to those skilled in the art may be used. In the present disclosure, the inhibitor targeting iFXase can target a terminal rate-limiting enzyme (iFXase) in the intrinsic coagulation pathway and may be used in the treatment of cardiovascular and cerebrovascular diseases and thrombotic diseases or drug development therefor.

The present disclosure further provides an anticoagulant drug and/or an antithrombotic drug targeting an intrinsic coagulation pathway, including: an active ingredient and a pharmaceutically acceptable auxiliary material, where the active ingredient is the HPS as described in above technical solutions or the HPS prepared by the preparation method as described above. In the present disclosure, there are no specific requirements for the pharmaceutically acceptable auxiliary material, and those well-known to those skilled in the art may be used. There are no specific requirements for a dosage form of the drug, and those well-known to those skilled in the art may be used. Specifically, in some embodiments, the dosage form is a tablet, a powder, a capsule, a granule, or a liquid preparation.

The present disclosure further provides a method for treating a vascular disease, including: administering the HPS, specifically administrating the inhibitor targeting intrinsic tenase (iFXase), or the anticoagulant drug and/or the antithrombotic drug for treatment. In some embodiments of the present disclosure, the vascular disease includes a thrombotic disease or a cardiovascular and cerebrovascular disease; a dosage of the anticoagulant drug and/or antithrombotic drug is preferably 10 mg/day/person to 30 mg/day/person; the thrombotic disease includes ischemic stroke; the HPS can specifically be used in the acute phase and recovery treatment phase of ischemic stroke.

The following clearly and completely describes the technical solutions of the present disclosure in conjunction with the examples. Apparently, the described examples are merely some rather than all of the examples of the present disclosure. All other examples obtained by a person of ordinary skill in the art based on the examples of the present disclosure without creative efforts shall fall within the scope of the present disclosure.

### Example 1 Extraction of HPS

The body walls of *Holothuria leucospilota* (Brandt) were carefully separated from other tissues and immediately immersed in acetone, and then stored at 4°C for 24 h. A dried body wall tissue (500 g) was ground into a powder and suspended in 10 L of 0.1 mol/L sodium acetate buffer (pH=6) containing 50 g of papain. A suspension was incubated at 65°C for 24 h. An incubation solution was then centrifuged at low temperature (4,000 rpm, 4°C for 30 min) to obtain an enzymatic hydrolysate supernatant. A 6 mol/L aqueous hydrochloric acid solution was added to the enzymatic hydrolysate supernatant to adjust the pH value to 2.5 ± 0.5. A resulting mixture was reacted for 2 h under stirring, followed by low-temperature centrifugation (4,000 rpm, 4°C for 30 min) to obtain an acid hydrolysate supernatant. A 40 wt% aqueous sodium hydroxide solution was added to the acid hydrolysate supernatant to adjust the pH value to 7.0 ± 0.5. Under stirring, 1-fold volume of 95 vol% ethanol (relative to the supernatant volume) was added to a resulting reaction system. A resulting mixture was maintained at -10°C for 24 h and then centrifuged at low temperature (4,000 rpm, 4°C for 30 min), and a formed precipitate was collected. The precipitate was dissolved in 500 mL of distilled water. 1 L of 95 vol% ethanol was then added thereto. A resulting mixture was maintained at -10°C for 24 h and then centrifuged at low temperature (4,000 rpm, 4°C for 30 min), and a formed precipitate was collected. The precipitate was washed with 500 mL of 95 vol% ethanol, centrifuged at low temperature (4,000 rpm, 4°C for 30 min), and a solid was collected. The solid was dissolved in 500 mL of distilled water and freeze-dried to obtain 4 g of crude HPS.

The 4 g of crude HPS was loaded onto a DEAE cellulose column (10 cm × 4 cm) pre-equilibrated with 0.1 mol/L HAc-NaAc buffer (pH=6). The column was successively rinsed with 5 L of HAc-NaAc buffer (pH=6) containing 0.5 mol/L NaCl and 2 L of HAc-NaAc buffer (pH=6) containing 1 mol/L NaCl, with a flow rate in the cellulose column of 50 mL/min, and 500 mL of per fraction eluted was collected. The fractions were analyzed using HPLC methods for molecular weight and molecular weight distribution. Fractions eluted of chromatographic peaks corresponding to a weight-average molecular weight between 90,000 and 130,000 were collected. 1-fold volume of 95 vol% ethanol (relative to an effluent volume) was added to collected fractions. A resulting mixture was maintained at -10°C for 24 h and then centrifuged at low temperature (4,000 rpm, 4°C for 30 min), and a formed precipitate was collected. The precipitate was dissolved in distilled water and concentrated to one-half the original volume using an ultrafiltration membrane with a molecular weight cut-off of 10,000. Water was added to restore the original volume, and a resulting solution was ultra-filtered again to one-half the original volume. Water was added to restore the original volume, and a resulting solution was ultra-filtered again to one-half the original volume. Water was added to restore the original volume, and a resulting solution was ultra-filtered again to one-half the original volume. A finally obtained concentrate was collected and freeze-dried to obtain 2.5 g of purified HPS (with the cation being sodium ion). Following the above procedure, 5 consecutive batch samples were prepared, designated as HPS batches 202401, 202402, 202403, 202404, and 202405.

### Example 2 Molecular Weight and Molecular Weight Distribution Test

The molecular weight and molecular weight distribution of the HPS obtained in Example 1 were tested using the HPLC method.

Chromatographic conditions: mobile phase A: 0.1 mol/L aqueous sodium sulfate solution; flow rate: 0.5 mL/min; elution condition: 100% A; chromatographic column: TSK Gel G4000PW xl (30 cm × 7.5 mm, 10 µm); column temperature: 35°C; refractive index (RI) detector temperature: 35°C.

Reference sample solution: dextran reference samples with molecular weights of 9750 Da, 13050 Da, 36800 Da, 64650 Da, 135350 Da, and 300600 Da were taken, dissolved and diluted with the mobile phase to prepare solutions with a concentration of 10 mg/mL.

Test sample solution: an appropriate amount of HPS from different batches (202401, 202402, 202403, 202404, and 202405) was accurately weighed, dissolved and diluted with the mobile phase to prepare 10 mg/mL test sample solutions.

Determination method: the molecular weight distribution of the HPS of different batches was determined according to the Size Exclusion Chromatography method (General Chapter 0514, Chinese Pharmacopoeia, Part IV). The results are shown in Table 1.

**Table 1 Molecular Weight Distribution Test Results**

| | | System precision | | | |
|---|---|---|---|---|---|
| Sample injection order | | | Peak area (mV×s) | | |
| 202401 | | | 1376565 | | |
| 202402 | | | 1380019 | | |
| 202403 | | | 1381909 | | |
| 202404 | | | 1384286 | | |
| 202405 | | | 1382562 | | |
| Peak area RSD (%) | | | 0.21 | | |

| Molecular weight data | | | | | |
|---|---|---|---|---|---|
| Name | Peak position | Weight-average molecular weight Mw | Number-average molecular weight Mn | Peak molecular weight Mp | Polydispersity index PDI |
| 202401 | 14.978 | 108791 | 82585 | 112717 | 1.32 |
| 202402 | 14.959 | 110089 | 84970 | 114340 | 1.30 |
| 202403 | 15.144 | 98522 | 74710 | 99682 | 1.32 |
| 202404 | 15.017 | 110014 | 86940 | 109465 | 1.27 |
| 202405 | 14.968 | 111832 | 88119 | 113614 | 1.27 |

According to the data in Table 1, it can be seen that the HPS of the present disclosure has a weight-average molecular weight of 90,000 to 130,000 and a polydispersity index (PDI) ≤ 1.32.

### Example 3 Monosaccharide Composition Analysis of HPS

### 1. Determination of Glucuronic Acid Content:

The glucuronic acid content in the HPS was tested using the m-hydroxydiphenyl colorimetric method.

Preparation of reference sample solution: 30 mg of glucuronic acid, dried to constant weight at 80°C, was accurately weighed, placed in a 50 mL volumetric flask, dissolved in water, and diluted to a volume reaching the mark, followed by shaking to be uniform. 5 mL of this solution was precisely taken, transferred to a 50 mL volumetric flask, diluted with water to a volume reaching the mark, followed by shaking to be uniform to obtain the reference sample solution (containing 60 µg of glucuronic acid per 1 mL).

Plotting of the standard curve: 0.00 mL, 0.10 mL, 0.20 mL, 0.30 mL, 0.40 mL, and 0.50 mL of the reference sample solution were accurately taken and placed separately into test tubes with stoppers, and water was added precisely to each test tube to make the volume 0.50 mL. In an ice bath, 3 mL of sodium tetraborate-sulfuric acid solution (prepared by dissolving 2.4 g of sodium tetraborate in 500 mL of sulfuric acid) was accurately added to each tube, followed by mixing well, and heated in a boiling water bath for 5 min. After taking out and cooling in an ice bath, to each test tube, 50 µL of m-hydroxydiphenyl solution (prepared by dissolving 75 mg of m-hydroxydiphenyl in 50 mL of 0.5% sodium hydroxide solution) was accurately added, followed by mixing well. The test tube containing the "0.00" mL reference sample solution was used as a blank control. Absorbance was measured at a wavelength of 520 nm according to the spectrophotometric method (reference: Pharmacopoeia of the People's Republic of China). A standard curve was plotted with absorbance as the ordinate and concentration as the abscissa.

Determination method: approximately 10 mg of the sample to be tested was accurately weighed, placed in a 50 mL volumetric flask, dissolved in water, and diluted to a volume reaching the mark, thereby obtaining a test solution. 0.50 mL of the test solution was accurately measured, and the absorbance was determined according to the method described in "Plotting of the standard" section, starting from "3 mL of sodium tetraborate-sulfuric acid solution was accurately added to each tube in an ice bath...". The glucuronic acid content in the test solution was read from the standard curve, and the glucuronic acid content in the sample to be tested was calculated.

### 2. Determination of Fucose Content:

Preparation of reference sample solution and test solutions: a sulfuric acid test solution was prepared by slowly adding 6 parts of concentrated sulfuric acid to 1 part of water (V/V), cooled, and set aside for use; a cysteine-phenol test solution containing 1.0 wt% L-cysteine hydrochloride and 0.075 wt% phenol was prepared; a 40 µg/mL fucose standard solution and a 50 µg/mL sample solution were prepared using distilled water.

Plotting of the standard curve: 0.00 mL, 0.05 mL, 0.10 mL, 0.15 mL, 0.20 mL, and 0.25 mL of the standard solution were added to test tubes with stoppers, respectively, and the volume in each tube was adjusted to 0.25 mL with distilled water. After cooling in an ice bath, 1.25 mL of the sulfuric acid test solution was added to each tube. The solutions in test tubes were mixed well and simultaneously immersed in a boiling water bath. The heating time was strictly controlled. After 3 min, the test tubes were taken out together, and transferred to an ice bath for cooling. 0.25 mL of the cysteine-phenol test solution was added to each tube. After mixing well, the test tubes were kept in the ice bath for 1 h. The test tube containing the "0.00" mL standard solution was used as a blank control. The light absorption at 398 nm was measured, and the standard curve was plotted.

Determination method: 0.25 mL of the sample solution was pipetted, and the rest of the procedure followed the method described in the "Plotting of the standard curve" section. Using the "0.00" test tube as a blank control, the light absorption at 398 nm was measured, and the fucose content was calculated according to the standard curve.

### 3. Determination of N-acetyl-D-galactosamine Content:

Preparation of reference sample solutions and test solutions: a 0.25 mol/L sodium tetraborate solution was prepared; a 3.5% (V/V) acetylacetone test solution was prepared using the sodium tetraborate solution; 0.16 g of *p*-dimethylaminobenzaldehyde (PDABA) was dissolved in 1.5 mL of 12 mol/L isopropanol, and diluted to prepare a PDABA test solution with a concentration of 0.1 g/mL for later use.

Plotting of standard curve and sample determination:
(1) Pretreatment of standard galactosamine and sample: 1.0 mg of galactosamine hydrochloride or an appropriate amount of sample (equivalent to 0.2 mg of galactosamine) was accurately weighed into ampoules. 6 mol/L hydrochloric acid was added at a ratio of 1 mg: 1 mL, the ampoules were sealed, and heated at 100°C for 3 h. After cooling, the acid was removed by evaporation at 70°C until dry. After drying, a small amount of distilled water was added thereto, and the acid removal process was repeated twice. The deacidified galactosamine was dissolved in distilled water at a ratio of 1 mg: 10 mL, which was used as the standard solution for the following determination test; the deacidified sample was dissolved in distilled water at a ratio of 1 mg: 4 mL to obtain the sample solution.
(2) Plotting of standard curve and determination of galactosamine in the sample: 0.00 mL, 0.10 mL, 0.20 mL, 0.30 mL, and 0.40 mL of the above standard solution were accurately pipetted into test tubes with stoppers, and the volume in each tube was adjusted to 0.40 mL with distilled water. 0.3 mL of the acetylacetone test solution was added, and the test tubes were incubated in a 25°C water bath for 2 h, followed by adding 1 mL of the PDABA test solution. After mixing, the test tubes were incubated at 50°C for 15 min. After leaving at room temperature for 30 min, the light absorption at 530 nm was measured, using the "0.00" tube as a blank control. The standard curve was plotted with concentration versus light absorption. Further, 0.4 mL of the sample solution was pipetted, and mixed with 0.3 mL of the acetylacetone test solution, and the procedure followed the method described in "Plotting of standard curve". Using the "0.00" tube as a blank control, the light absorption was measured, and the galactosamine content in the sample was determined according to the standard curve (NOTE: the standard sample should be converted from galactosamine hydrochloride to *N*-acetyl-*D*-galactosamine).

### 4. Test Results:

The monosaccharide composition test results for various batches of HPS samples are shown in Table 2.

**Table 2 Monosaccharide composition results of HPS (mass fraction)**

| Batch No. | 202401 | 202402 | 202403 | 202404 | 202405 |
|---|---|---|---|---|---|
| Glucuronic acid content | 22.40% | 21.68% | 21.58% | 20.75% | 19.71% |
| *N-*acetyl*-D-*galactosamine content | 26.42% | 26.39% | 23.55% | 22.70% | 24.04% |
| Fucose content | 12.78% | 12.70% | 11.97% | 12.22% | 10.35% |

Based on 100 g of HPS, the molar contents of monosaccharides were calculated according to Table 2. The results are shown in Table 3.

**Table 3 Molar content of monosaccharides in HPS (calculated based on 100 g of HPS)**

| Batch No. | 202401 | 202402 | 202403 | 202404 | 202405 | Average |
|---|---|---|---|---|---|---|
| Glucuronic acid (mol) | 0.115 | 0.112 | 0.111 | 0.107 | 0.102 | 0.109 |
| *N*-acetyl-*D*-galactosamine (mol) | 0.119 | 0.119 | 0.106 | 0.103 | 0.109 | 0.111 |
| Fucose (mol) | 0.078 | 0.077 | 0.073 | 0.0745304 055 | 0.063 | 0.073 |

The molar ratios among monosaccharides in the HPS were calculated based on Table 3. The results are shown in Table 4.

**Table 4 Molar ratios of monosaccharide components in HPS**

| Batch No. | 202401 | 202402 | 202403 | 202404 | 202405 | Average |
|---|---|---|---|---|---|---|
| Glucuronic acid (mol) | 1 | 1 | 1 | 1 | 1 | 1 |
| *N*-acetyl-*D*-galactosamine (mol) | 1.04 | 1.06 | 0.96 | 0.96 | 1.07 | 1.02 |
| Fucose (mol) | 0.67 | 0.69 | 0.66 | 0.7 | 0.62 | 0.67 |

According to the results in Table 4, a molar ratio of glucuronic acid, *N*-acetyl-*D*-galactosamine, and fucose in the HPS of the present disclosure was 1 : 0.9-1.1 : 0.6-0.7.

### Example 4 Determination of Sodium Ion Content in HPS

### 1. Instrument and Method

The sodium ion content in the HPS was tested using ion chromatography. The instrument and method are shown in Table 5.

**Table 5 Sample, instrument, and chromatographic system information for sodium ion content determination**

| Samples | 202401-202405 | | | |
|---|---|---|---|---|
| Reference sample | Name | Source | Batch No. | Purity |
| | Sodium chloride | Tianjin Damao Chemical Reagent Factory, China | 20230401 | 99.8% |
| Chromatographic column | Chromatographic column: OR-M-2 (No.: 0620240820-62) | | | |
| Ion chromatograph | | Ourun/OIC-610 | | |
| Mobile phase | | Mobile phase: 7.5 mM methanesulfonic acid (MSA) | | |
| | | Flow rate: 1.0 mL/min; | | |
| | | Injection volume: 15 µL; | | |
| Chromatographic conditions | | Column temperature: 40°C; | | |
| | | Detector temperature: 30°C | | |
| | | Solvent: water | | |
| | | Suppressor current: 70 mA | | |
| | | Measurement time: 30 min | | |

### 2. Content Determination

Preparation of reference sample solution: 2.54159 g of sodium chloride was weighed into a 100 mL volumetric flask, dissolved in water, and diluted to a volume reaching the mark to prepare a 10,000 µg/mL stock solution; 0.5 mL of this stock solution was pipetted into a 50 mL volumetric flask, and diluted with water to a volume reaching the mark, to prepare a 100 µg/mL working solution; 0.125 mL, 0.25 mL, 0.5 mL, 1.25 mL, 2.5 mL, 3.75 mL, and 5 mL of the working solution were accurately taken into 7 separate 25 mL volumetric flasks, and diluted with water to a volume reaching the mark, to prepare sodium ion standard solutions with concentrations of 0.5 µg/mL, 1 µg/mL, 2 µg/mL, 5 µg/mL, 10 µg/mL, 15 µg/mL, and 20 µg/mL, respectively.

Preparation of test sample solution: 10.00 mg of the test sample was weighed into a 10 mL volumetric flask, dissolved with purified water, diluted with purified water to a volume reaching the mark, and mixed well to obtain the test sample stock solution; 1.0 mL of the test sample stock solution was accurately transferred to a 10 mL volumetric flask, diluted with water to a volume reaching the mark, and a resulting mixture was filtered to obtain the test sample solution (with a concentration of 99.756 µg/mL).

Linear experiment results: the series of sodium ion standard solutions with concentrations of 0.5 µg/mL, 1 µg/mL, 2 µg/mL, 5 µg/mL, 10 µg/mL, 15 µg/mL, and 20 µg/mL were injected and analyzed, respectively. The results obtained are shown in Table 6.

**Table 6 Sodium ion linearity test results**

| Sample | Concentration (µg/mL) | Peak area |
|---|---|---|
| 5% | 0.5001 | 389.2919 |
| 10% | 1.000 | 564.9002 |
| 25% | 2.001 | 1304.71739 |
| 50% | 5.001 | 2761.19062 |
| 100% | 10.00 | 5513.44555 |
| 150% | 15.00 | 8139.68196 |
| 200% | 20.01 | 10806.76113 |

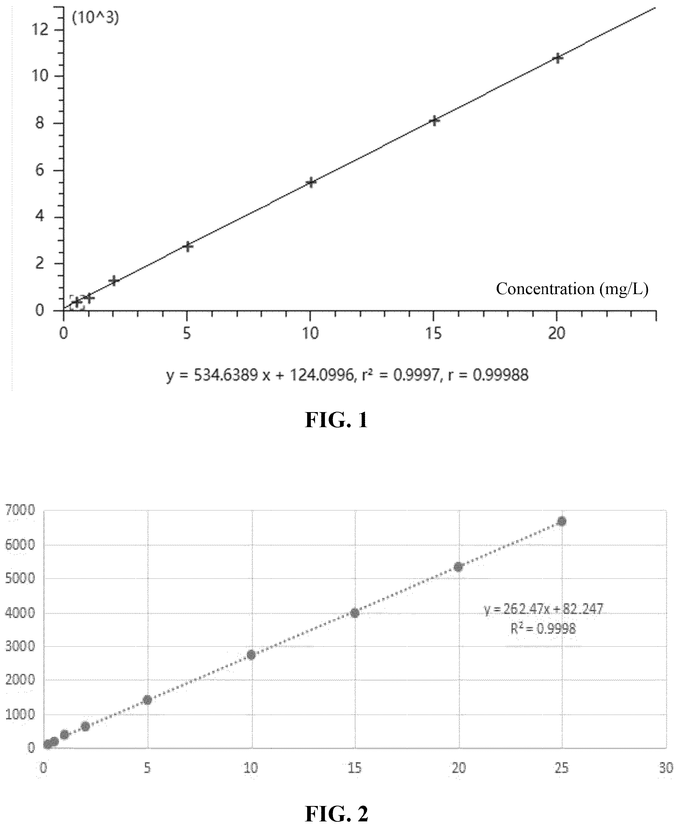

Linear regression analysis was conducted using the mass concentration of the analyte (X, µg·mL⁻¹) as the abscissa and the peak area (Y) as the ordinate. The linear regression analysis result is shown in FIG. 1. The sodium ion regression equation was: Y = 534.6389x + 124.0996, with a correlation coefficient r = 0.99988.

The above results indicated that the sodium ion showed a desirable linear relationship within the range of 0.5001-20.01 µg/mL, meeting the validation protocol requirements (linearity range: at least 5% to 200% of the injection concentration). Within this range, the linear regression coefficient R₂ was 0.99997, which also met the validation protocol requirements (regression coefficient (R²) ≥ 0.9990).

### 3. Content Determination Results

Test samples from different batches were accurately weighed, and prepared into test sample solutions according to the method described in "Preparation of test sample solution" section. An appropriate amount of the Na⁺ ion reference sample was accurately weighed, and two reference sample solutions were prepared in parallel according to the method described in "Preparation of reference sample solution" section, labeled as Control-1 and Control-2. The reference sample solutions were used for system suitability testing, where Control-1 was injected 5 times repeatedly, and Control-2 was injected 2 times repeatedly. 15 µL each of a mixed reference sample solution and the test sample solutions were accurately injected into the ion chromatograph, the chromatograms were recorded, and the Na⁺ ion content was calculated using the external standard method. System suitability test results for the assay are shown in Table 7 below, and the test results of the samples are shown in Table 8.

**Table 7 System suitability test results for the assay**

| System suitability in content determination | | | | | |
|---|---|---|---|---|---|
| Name | Concentration (µg/mL) | No. | Peak area | Peak area RSD (%) | Retention time (min) |
| Control -1 | 5.018 | 1 | 2334.248 | 0.30 | 14.635 |
| | | 2 | 2318.49 | | 14.641 |
| | | 3 | 2329.858 | | 14.632 |
| | | 4 | 2320.127 | | 14.627 |
| | | 5 | 2320.482 | | 14.628 |
| Control -2 | 5.063 | 1 | 2433.67 | / | 14.627 |
| | | 2 | 2423.055 | | 14.632 |
| Conclusion: after repeated 5 injections of the control solution, the RSD% of the Na⁺ ion peak area of 5 repeated injections of the reference solution is (0.30%), meeting the requirement (RSD ≤ 2.0%) | | | | | |

**Table 8 Sodium ion content determination test results**

| Solution No. | Sample injection amount (g) | Dilution factor | Concentration (µg/mL) | Peak area | Actually determined content (g/g) | Mass percentage (%) |
|---|---|---|---|---|---|---|
| 202401 | 0.01006 | | 100.6 | 4348.579 | 0.0924 | 9.24 |
| 202402 | 0.01005 | | 100.5 | 4263.765 | 0.0907 | 9.07 |
| 202403 | 0.01004 | 100 | 100.4 | 4457.325 | 0.0949 | 9.49 |
| 202404 | 0.01003 | | 100.3 | 4174.691 | 0.0889 | 8.89 |
| 202405 | 0.01009 | | 100.9 | 4746.989 | 0.1006 | 10.06 |

The above results indicated that the mass percentage of the sodium ion in the HPS of the present disclosure was within the range of 9% to 11%.

### Example 5 Determination of Sulfate Group Content in HPS

The sulfate group content (in term of sulfate ion) can be determined by ion chromatography and colorimetric method. The determined sulfate group content may vary depending on the method used. A specific process was as follows:

Method I: Determination of Polysaccharide Sulfate Group Content by Ion Chromatography

### 1. Instrument and Method

The sulfate group content in the HPS was tested using ion chromatography. The instrument and method are shown in Table 9.

**Table 9 Instrument and chromatographic system information table for sulfate group content determination method**

| Test solution | 202401-202405 | | | |
|---|---|---|---|---|
| Reference sample | Name | Source | Batch No. | Content |
| | Sulfate standard solution | Macklin | C16330579 | 100 µg/mL |
| Chromatographic column | Chromatographic column: Shodex/IC SI-904E (No.: G1490040) | | | |
| Ion chromatograph | | | Ourun/OIC-610 | |
| Mobile phase | | | Mobile phase: 1.8 mM anhydrous sodium carbonate + 1.7 mM sodium bicarbonate | |
| | | | Flow rate: 1.0 mL/min; | |
| | | | Injection volume: 25 µL; | |
| Chromatographic conditions | | | Column temperature: 25°C; | |
| | | | Detector temperature: 30°C | |
| | | | Solvent: mobile phase | |
| | | | Suppressor current: 40 mA | |
| | | | Detection time: 20 min | |

### 2. Content Determination

Preparation of reference sample solution: 0.02 mL, 0.05 mL, 0.1 mL, 0.2 mL, 0.5 mL, 1 mL, 1.5 mL, 2 mL, and 2.5 mL of the sulfate ion stock solution (100 µg/mL) were accurately taken into 9 separate 10 mL volumetric flasks, diluted with water to a volume reaching the mark, thereby preparing sulfate ion standard solutions with concentrations of 0.2 µg/mL, 0.5 µg/mL, 1 µg/mL, 2 µg/mL, 5 µg/mL, 10 µg/mL, 15 µg/mL, 20 µg/mL, and 25 µg/mL, respectively.

Preparation of test sample solution: approximately 30.00 mg of the test sample was accurately weighed into a 20 mL volumetric flask, 15 mL of 2 mol/L hydrochloric acid was added thereto, and a resulting mixture was heated in a sealing condition at 90°C for 2 h. The hydrochloric acid therein was removed under reduced pressure, and a resulting residue was dissolved and diluted with the mobile phase to a marked volume in a 50 mL volumetric flask. 1 mL of a resulting solution was accurately transferred to a 20 mL volumetric flask, diluted with the mobile phase to a volume reaching the mark, mixed well, and filtered to obtain the test sample solution (with a concentration of 0.03 mg/mL).

### Linearity Experiment Results

The series of sulfate ion standard solutions with concentrations of 0.2 µg/mL, 0.5 µg/mL, 1 µg/mL, 2 µg/mL, 5 µg/mL, 10 µg/mL, 15 µg/mL, 20 µg/mL, and 25 µg/mL were injected and analyzed, respectively. The results obtained are shown in Table 10.

**Table 10 Sulfate ion linearity test**

| Test solution | Concentration (µg/mL) | Peak area |
|---|---|---|
| 2% | 0.2 | 102.3833 |
| 5% | 0.5 | 183.08611 |
| 10% | 1 | 385.69977 |
| 20% | 2 | 628.64865 |
| 50% | 5 | 1406.25683 |
| 100% | 10 | 2731.11205 |
| 150% | 15 | 3970.69577 |
| 200% | 20 | 5323.54837 |
| 250% | 25 | 6665.06959 |

Linear regression analysis was conducted using the mass concentration of the analyte (X, µg·mL⁻¹) as the abscissa and the peak area (Y) as the ordinate. The result is shown in FIG. 2. The sulfate ion regression equation was Y = 262.47X + 82.247, with a correlation coefficient r = 0.9998.

The above results indicated that the sulfate ion showed a desirable linear relationship within the range of 0.201-25.144 µg/mL, meeting the validation protocol requirements (linearity range: at least 5% to 200% of the injection concentration). Within this range, the linear regression coefficient R² was 0.9998, which also met the validation protocol requirements (regression coefficient (R²) ≥ 0.9990).

### 3. Content Determination Results

Test samples from different batches were accurately weighed, and test sample solutions were prepared according to the method described in "Preparation of test sample solution" section. An appropriate amount of the sulfate ion reference sample was accurately weighed, and two reference sample solutions were prepared in parallel according to the method described in "Preparation of reference sample solution", labeled as Control-1 and Control-2. The reference sample solutions were used for system suitability testing, where Control-1 was injected 5 times repeatedly, and Control-2 was injected 2 times repeatedly. 25 µL each of a mixed reference sample solution and the test sample solutions were accurately injected into the ion chromatograph, the chromatograms were recorded, and the sulfate ion content was calculated using the external standard method. The test results of the system suitability solution for content determination are shown in Table 11 below, and the test results of the samples are shown in Table 12.

**Table 11 Test results of system suitability solution for content determination**

| System suitability in content determination | | | | |
|---|---|---|---|---|
| Name | Concentration (µg/mL) | No. | Peak area | Peak area RSD (%) |
| Control-1 | 10.00 | 1 | 2804.03 | 0.39 |
| | | 2 | 2821.85 | |
| | | 3 | 2802.983 | |
| | | 4 | 2792.385 | |
| | | 5 | 2811.03 | |
| Control-2 | 10.100 | 1 | 2858.745 | / |
| | | 2 | 2833.763 | |
| Conclusion: after repeated 5 injections of the control solution, the RSD% of the SO₄ ion peak area of 5 repeated injections of the reference solution is (0.39%), meeting the requirement (RSD ≤ 2.0%) | | | | |

**Table 12 Test results for sulfate group content determination**

| Solution No. | Sample injection amount (g) | Dilution factor | Concentration (µg/mL) | Peak area | Actually determined concentration (µg/mL) | Content (%) |
|---|---|---|---|---|---|---|
| 202401 | 0.02941 | | 29.41 | 2227.181 | 8.0283 | 27.30 |
| 202402 | 0.03017 | | 30.17 | 2420.437 | 8.7249 | 28.92 |
| 202403 | 0.03135 | 1000 | 31.35 | 2699.342 | 9.7302 | 31.04 |
| 202404 | 0.03043 | | 30.43 | 2744.916 | 9.8945 | 32.52 |
| 202405 | 0.03013 | | 30.13 | 2810.229 | 10.1299 | 33.62 |

The above results indicated that the mass percentage of the sulfate groups in the HPS of the present disclosure was within the range of 26% to 35%.

### Method II: Determination of Sulfate Group Content by Terho Method

### Preparation of reference sample solution and test solutions:

A barium chloride buffer was prepared by mixing 10 mL of 2 mol/L hydrochloric acid solution, 2 mL of 0.005 mol/L barium chloride solution, and 8 mL of 0.02 mol/L sodium bicarbonate solution, then adding absolute ethanol to a total volume of 100 mL. A sodium rhodizonate solution was prepared by dissolving 5 mg of sodium rhodizonate in 20 mL of water, then adding 100 mg of ascorbic acid, mixing well, and adding ethanol to a final volume of 100 mL; this solution was prepared immediately before use. 36.29 mg of potassium sulfate (dried to constant weight at a temperature of 105°C to 110°C) was accurately weighed, placed in a 100 mL volumetric flask, dissolved in water, and diluted with water to a volume reaching the mark to prepare a stock solution. 1 mL of the stock solution was taken into a 10 mL volumetric flask and diluted with water to a volume reaching the mark, to obtain the standard sulfate group solution (1 mL equivalent to 20 µg of sulfate group). 1 mg of the sample was accurately weighed into a 2 mL ampoule. The sample was dissolved in 1.0 mL of 2 mol/L hydrochloric acid per 1.0 mg of sample. The ampoule was heated at 100°C for 1 h. After cooling, the solution was evaporated to dryness under reduced pressure at 65°C. A small amount of water was added, and the evaporation under reduced pressure was repeated to remove residual acid. The hydrolysis residue was dissolved in water at a ratio of 1 mg: 4 mL to serve as the test sample solution for sulfate group determination.

Plotting of the standard curve: 0, 0.1, 0.2, 0.3, 0.4, and 0.5 mL of the standard sulfate group solution were accurately pipetted into separate test tubes with stoppers. Water was added to each test tube to bring the volume to 0.5 mL, followed by adding 2 mL of ethanol and 1 mL of the barium chloride buffer sequentially. After mixing, 1.5 mL of the sodium rhodizonate test solution was added thereto. The test tubes were mixed well, left to stand for 10 min, and the light absorption at 510 nm was measured, using the test tube containing "0.00" mL standard solution as a blank control. A standard curve was plotted with concentration versus light absorption value.

Determination method: 0.1 mL of the sample solution was pipetted, and water was added to bring the volume to 0.5 mL. Other steps were the same as those described in "Plotting of the standard curve" section, and the light absorption was measured. The total sulfate group content was determined according to the standard curve.

### Test results:

The sulfate group content determination results for various batches of HPS samples are shown in Table 13.

**Table 13 Sulfate group content determination results of HPS (mass fraction)**

| Batch No. | 202401 | 202402 | 202403 | 202404 | 202405 |
|---|---|---|---|---|---|
| Sulfate group content | 33.34% | 34.52% | 36.74 | 38.23 | 39.05% |

The above results indicated that, as determined by the colorimetric method, the mass percentage of the sulfate groups in the HPS of the present disclosure was within the range of 32% to 40%.

### Example 6 Structural Identification

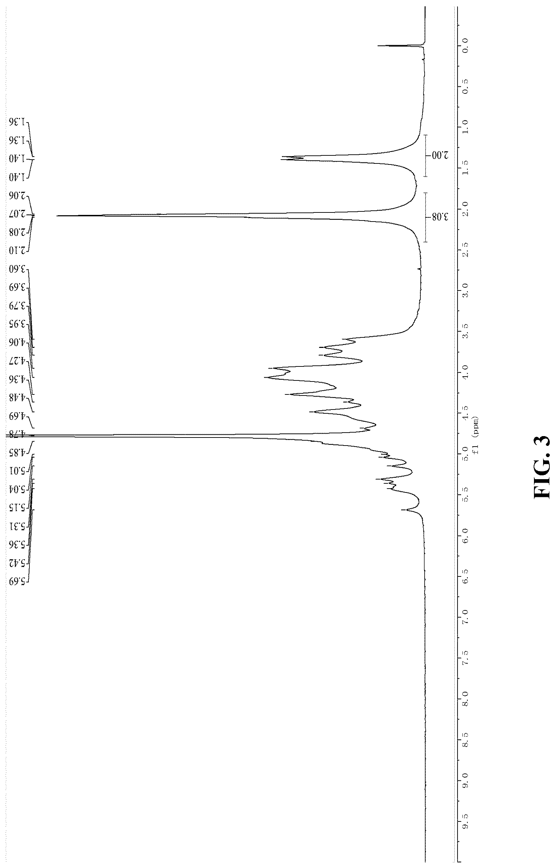

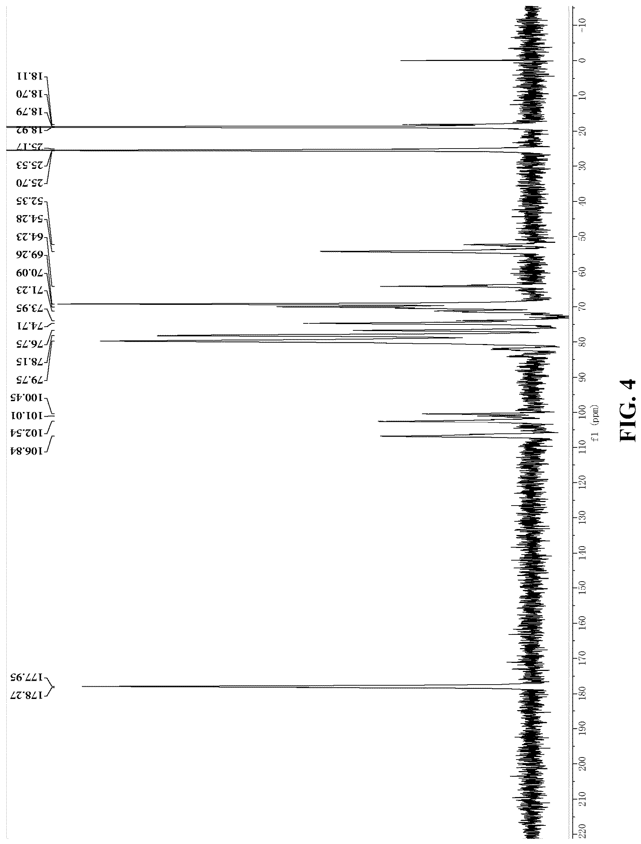

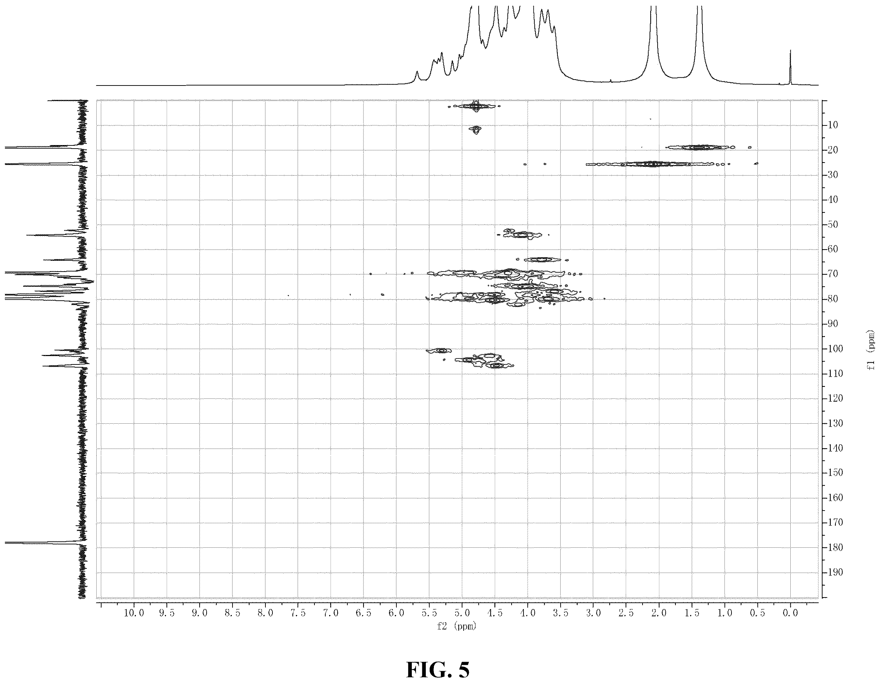

20 mg of HPS (Batch 202401) was weighed and dissolved in 1 mL of deuterium oxide. Using sodium 3-(trimethylsilyl)propionate-2,2,3,3-d4 as the internal standard (δ*_{H}*, 0 ppm), the ¹H NMR, ¹³C NMR, and HSQC spectra of the HPS were tested using a Bruker 600 MHz nuclear magnetic resonance spectrometer. The results are shown in FIG. 3 to FIG. 5.

The structural characteristics were analyzed according to literature data in combination with typical signal peaks. In the ¹H NMR spectrum, anomeric proton signals of fucose moieties with sulfate group modifications at different positions (or without sulfate group modification) appear at δ*_{H}* 5.70 ppm to 4.90 ppm. Combined with the HSQC spectrum, the signal peaks appearing at δ*_{H}* 4.80 ppm to 4.45 ppm include anomeric proton signals of glucuronic acid moieties with sulfate group modifications at different positions (or without sulfate group modification). Combined with the HSQC spectrum, the signal peaks appearing at δ*_{H}* 4.90 ppm to 4.50 ppm include anomeric proton signals of *N*-acetyl-*D*-galactosamine moieties with sulfate group modifications at different positions (or without sulfate group modification). Further, acetyl proton signal peaks of *N*-acetyl-*D*-galactosamine moieties appear at δ*_{H}* 2.40 ppm to 1.80 ppm; methyl proton signal peaks of fucose moieties appear at δ*_{H}* 1.60 ppm to 1.10 ppm. In the ¹³C NMR spectrum, anomeric carbon signal peaks of glucuronic acid moieties appear at δ*_{C}* 107.0 ppm to 105.0 ppm; anomeric carbon signal peaks of *N*-acetyl-*D*-galactosamine moieties appear at δ*_{C}* 103.0 ppm to 101.0 ppm; anomeric carbon signal peaks of fucose moieties appear at δ*_{C}* 104.0 ppm to 103.0 ppm and δ*_{C}* 101.0 ppm to 98.0 ppm; 2-position carbon (N-CH₂-) signal peaks of *N*-acetyl-*D*-galactosamine moieties appeared at δ*_{C}* 56.0 ppm to 51.0 ppm; acetyl carbon signal peaks of *N*-acetyl-*D*-galactosamine moieties appear at δ*_{C}* 27.0 ppm to 24.0 ppm; methyl carbon signal peaks of fucose moieties appear at δ*_{C}* 20.0 ppm to 16.0 ppm.

Based on the NMR spectroscopic information, the HPS of the present disclosure belongs to a fucosylated chondroitin sulfate component derived from sea cucumber. The backbone of this type of component is a copolymer of disaccharide units composed of *N*-acetyl-*D*-galactosamine and glucuronic acid. The structural differences lie only in the content and modification sites of the fucosyl and sulfate groups, as well as the molecular weight distribution.

Accordingly, the structural formula of the HPS prepared in Example 1 is as follows: 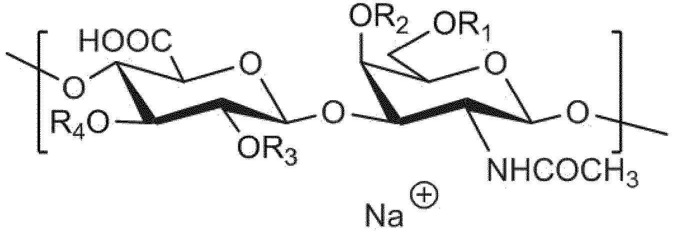 where R₁ is selected from the group consisting of H, SO₃⁻, and fucosyl; R₂ is selected from the group consisting of H and SO₃⁻; R₃ is selected from the group consisting of H and SO₃⁻; R₄ is selected from the group consisting of H and fucosyl; at least one of the R₁ and the R₄ is fucosyl; and X^{⊕} represents a cation; the fucosyl group has a structure as follows: 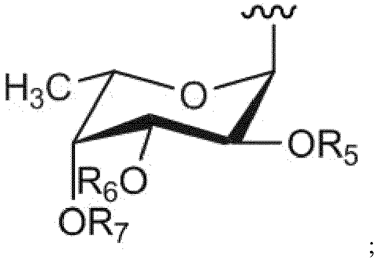 where R₅ is selected from the group consisting of H and SO₃⁻; R₆ is selected from the group consisting of H and SO₃⁻; and R₇ is selected from the group consisting of H and SO₃⁻.

From results of Examples 2 to 5, the HPS of the present disclosure has a weight-average molecular weight of 90,000 to 130,000 (PDI < 1.32), where the molar ratio of glucuronic acid, N-acetyl-D-galactosamine, and fucose is 1 : 0.9-1.1 : 0.6-0.7, the mass percentage of the sodium ion is 9% to 11%, and the mass percentage of the sulfate groups is 25% to 40%. This compound is a novel fucosylated chondroitin sulfate polysaccharide. Furthermore, the polysaccharide having the specific structure described above can target the terminal rate-limiting enzyme (intrinsic tenase, iFXase) in the intrinsic coagulation pathway, with high activity, and achieves the effect of anticoagulation without bleeding. This compound holds broad prospects in the development of anticoagulant drugs targeting the rate-limiting enzyme of the intrinsic coagulation pathway.

### Example 7 Anticoagulant and Antithrombotic Experiments in Mice

### 1. Experimental materials

Experimental animals: 24 mice (C57BL/6 mice) with uniform body weight.

Test substance: HPS prepared in Example 1 (Batch number: 202401).

### 2. Experimental Principle

Different doses of HPS (DT-1) were injected into mice. Plasma was collected from the mice 1 h later, and coagulation function indicators such as PT, APTT, and TT were measured to determine the effect of the test substance on the coagulation function of the mice.

### 3. Experimental Methods

### 3.1 Animal Grouping

Random grouping: after animal reception, the mice were acclimatized for 5-7 d. During the acclimatization period, the mice were observed for appearance and general condition. Only mice that passed through the inspection were used in this experiment. After the acclimatization period, the body weights of the mice were measured, and the mice were randomly divided into a blank control group and test substance groups (with low, medium, and high doses).

### 3.2 Administration Period

Mice in the successfully grouped test substance groups (with low, medium, high doses) were administered with the test substance via tail vein injection. In a blank control group, a corresponding dose of physiological saline was administrated. The administration amount for each group are shown in Table 14.

**Table 14 Administration information for animals in each group**

| Group | Administration mode | Dose | Number of C57BL/6 mice |
|---|---|---|---|
| Blank control | Tail vein injection | Equal amount of physiological saline | 6 mice |
| Low-dose test substance group | Tail vein injection | 2 mg/kg body weight | 6 mice |
| Medium-dose test substance group | Tail vein injection | 4 mg/kg body weight | 6 mice |
| High-dose test substance group | Tail vein injection | 8 mg/kg body weight | 6 mice |

### 3.3 Observation Period

General vital signs were observed during the experiment.

### 3.4 Main Test Indicators

Plasma PT, APTT, and TT levels were tested.

### 4. Experimental Data and Results

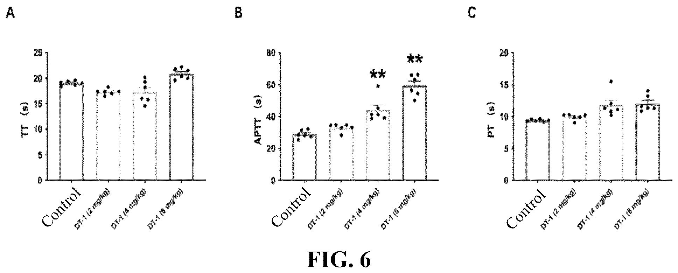

The results are shown in FIG. 6. The results in FIG. 6 indicate that 1 h after administration via tail vein injection, compared with the mice in the blank control group, the test substance groups show no significant changes in plasma PT and TT levels, but the APTT level in the administration groups showed a dose-dependent and significant increase (**P < 0.01 vs. Control). It is concluded that the HPS exerts its anticoagulant effect by inhibiting the intrinsic coagulation pathway.

### Example 8 Effect of HPS on Venous Thrombosis in Mice

### 1. Experimental materials

Experimental animals: 24 mice (Km mice) with uniform body weight.

Test substance: HPS prepared in Example 1 (Batch number: 202401).

### 2. Experimental Principle

A mouse model of tail thrombosis was established by tail vein injection of carrageenan, which induced thrombus formation after 24 h. 30 min before this, the test substance was administered to mice via tail vein injection, to determine its effect on thrombus formation.

### 3. Experimental Methods

### 3.1 Animal Grouping

Random grouping: after animal reception, the mice were acclimatized for 5-7 d. During the acclimatization period, the mice were observed for appearance and general condition. Only mice that passed through the inspection were used in this experiment. After the acclimatization period, Km mice were weighed randomly and randomly divided into 4 groups: the tail thrombosis model group, and HPS (DT-1) groups of different doses (namely, low dose, medium dose, and high dose).

### 3.2 Establishment of the Tail Thrombosis Model

The mouse tail thrombosis model was established by tail vein injection of carrageenan. Healthy male Km mice (22 ± 1 g) were injected with carrageenan (1 mg/kg) via the tail vein to induce thrombosis for 24 h.

### 3.3 Administrating and Modeling Period

Km mice in the successfully grouped test substance (low dose, medium dose, high dose) groups were administered DT-1 via tail vein injection 30 min before carrageenan induction. Tail thrombosis indicators were measured 24 h later. The administration amount for each group is shown in Table 15.

**Table 15 Administration information for mice in each group**

| Group | Administration mode | Dose | Number of Km mice |
|---|---|---|---|
| Model group | No administration | None | 5 mice |
| Low-dose test substance group | Tail vein injection | 0.2 mg/kg body weight | 5 mice |
| Medium-dose test substance group | Tail vein injection | 0.4 mg/kg body weight | 5 mice |
| High-dose test substance group | Tail vein injection | 0.8 mg/kg body weight | 5 mice |

### 3.4 Observation Period

General vital signs were observed during the experiment.

### 3.5 Main test Indicators

The length of the thrombus formed in the mouse tail and its percentage relative to the total tail length were observed.

### 4. Experimental Data and Results

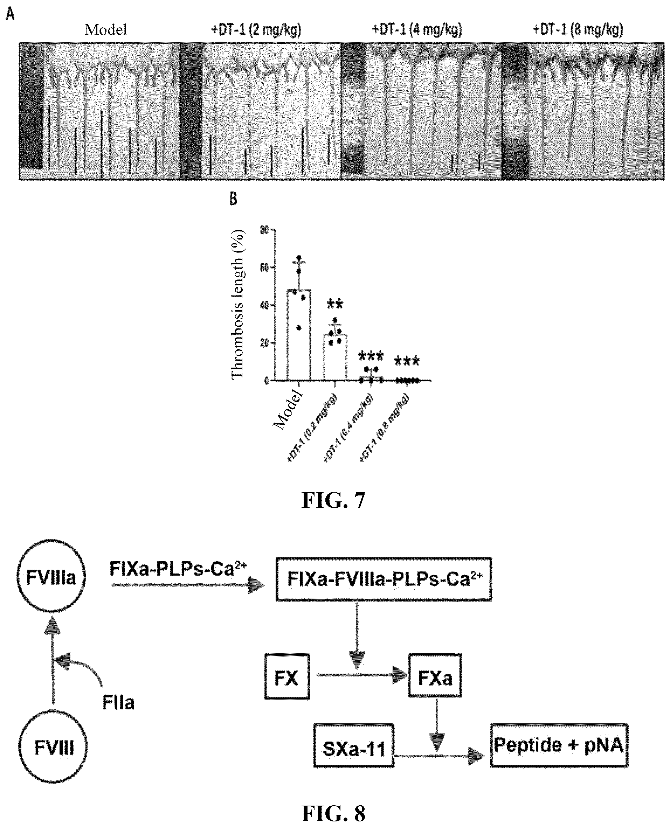

The results are shown in FIG. 7. In FIG. 7, panel A shows images of the thrombus length in mouse tails, and panel B shows a percentage of the length of the tail thrombus relative to the total tail length. The results in FIG. 7 indicate that the model group exhibits significant tail thrombosis. Compared with the model group, the percentage of the length of tail thrombus in the HPS (DT-1) drug groups decreases in a dose-dependent manner (**P < 0.01 *vs.* Model, ***P *< 0.001 vs.* Model).

### Example 9 Effect of HPS in Inhibiting Coagulation Factors IIa and Xa Activities

### 1. Test Objective

The pharmacological effect of HPS is that of an anticoagulant drug. This example studied effect of HPS in IIa, Xa, ATIII-IIa, and ATIII-Xa activities. The activities of the aforementioned coagulation factors were tested with reference to the heparin bioassay method.

### 2. Instruments and Materials

The instruments required for the experiment are listed in Table 16, and the test drugs and kits required are listed in Table 17.

**Table 16 Instruments Required for the Experiment**

| Name | Model/specification | Manufacturer |
|---|---|---|
| Microplate reader | BioTeK SynergyH1 | Agilent |
| Pipette | 1000 µL | Eppendorf |
| Pipette | 200 µL | Eppendorf |
| Pipette | 20 µL | Eppendorf |
| Pipette | 10-100 µL eight-channel pipette | Eppendorf |
| Electronic multi-channel dispensing pipette | 1 µL-50 mL Multipette^{®}E3x | Eppendorf |
| Electronic balance | ME104T/02 | Mettler |
| Thermostatic water bath | WB20 | SalvisLAB |

**Table 17 Test Drugs and Kits Required for the Experiment**

| Name | Batch No. | Trait | Specification | Source |
|---|---|---|---|---|
| HPS | 202401 | Freeze-dried powder | / | Harbin Hongdoushan Bio-pharm Co., Ltd., China |
| Human coagulation factor IIa | 140625-202215 | Freeze-dried powder | 680 IU/bottle | China National Institutes for Food and Drug Control |
| Human coagulation factor Xa | 140826-202102 | Freeze-dried powder | 46 nkat/bottle | China National Institutes for Food and Drug Control |
| ATIII | 140825-202303 | Freeze-dried powder | 10 IU/bottle | China National Institutes for Food and Drug Control |
| S-2238 | 140835-202102 | Freeze-dried powder | 18.8 mg/bottle | China National Institutes for Food and Drug Control |
| S-2765 | 140834-202102 | Freeze-dried powder | 21.5 mg/bottle | China National Institutes for Food and Drug Control |

In Table 17, S-2238 and S-2765 were chromogenic substrates.

### 3. Experimental Methods

### 3.1 Preparation of TRIS Buffer (pH=8.4)

0.606 g of tris(hydroxymethyl)aminomethane, 1.23 g of sodium chloride, 0.28 g of disodium ethylenediaminetetraacetate, and 0.1 g of polyethylene glycol 6000 were taken, dissolved in 80 mL of water, the pH value of a resulting solution was adjusted to 8.4 with hydrochloric acid, and a resulting mixture was diluted with water to 100 mL.

### 3.2 Preparation of Standard Solution and Chromogenic Substrate Solutions

(1) Preparation of IIa solution: on the day of the experiment, 1 mL of TRIS buffer was pipetted and added to a IIa standard, thereby preparing a 680 IU/mL IIa standard stock solution. 5 µL of the above stock solution was taken, diluted to 1 mL with TRIS buffer, and mixed well to obtain a 3.4 IU/mL IIa solution.
(2) Preparation of Xa solution: on the day of the experiment, TRIS buffer was pipetted and added to the Xa standard to prepare a 7.66 nkat/mL Xa standard solution.
(3) Preparation of chromogenic substrate S-2238 solution: S-2238 was dissolved in water to prepare a 0.003 mol/L stock solution, which was diluted with water to 0.6 mmol/L before use.
(4) Preparation of chromogenic substrate S-2765 solution: S-2765 was dissolved in water to prepare a 0.003 mol/L stock solution, which was diluted with water to 1 mmol/L before use.
(5) Preparation of antithrombin (ATIII) solution: Tris buffer (pH=8.4) was added to ATIII standard, and the ATIII standard was dissolved, followed by diluting with the Tris buffer to prepare a solution containing 0.15 IU/mL of antithrombin per 1 mL.
(6) Preparation of polysaccharide working solutions: 25 mg of HPS was taken and 10 mL of Tris buffer was added thereto to obtain Working Solution A with a concentration of 2.5 mg/mL; 200 µL of Working Solution A was taken, and 800 µL of Tris buffer was added thereto to obtain Working Solution B with a concentration of 500 µg/mL; 200 µL of Working Solution B was taken, and 800 µL of Tris buffer was added thereto to obtain Working Solution C with a concentration of 100 µg/mL; 200 µL of Working Solution C was taken and 800 µL of Tris buffer was added thereto to obtain Working Solution D with a concentration of 20 µg/mL; 200 µL of Working Solution D was taken and 800 µL of Tris buffer was added thereto to obtain Working Solution E with a concentration of 4 µg/mL; 200 µL of Working Solution E was taken and 800 µL of Tris buffer was added thereto to obtain Working Solution F with a concentration of 0.8 µg/mL.
(7) Preparation of reaction stopping solution: 1 g of citric acid was weighed and added to 50 mL of ddH₂O to prepare a 2% citric acid stopping solution.

### 4. Polysaccharide Activity Determination

4.1 IIa Activity Test: 20 µL of Tris buffer and 20 µL of polysaccharide working solutions at different concentrations were added to each well (20 µL of Tris buffer was added to the blank well instead of the polysaccharide working solution), followed by adding 40 µL of IIa solution, and incubation was conducted at 37°C for 15 min; 40 µL of 0.6 mmol/L S-2238 solution was added, incubation was further conducted in a 37°C water bath for 15 min, and 40 µL of the reaction stopping solution was then added thereto, and the absorbance at 405 nm was measured.

4.2 Xa Activity Test: 20 µL of Tris buffer and 20 µL of polysaccharide working solutions at different concentrations were added to each well (20 µL of Tris buffer was added to the blank well instead of the polysaccharide working solution), followed by adding 40 µL of Xa solution, and incubation was conducted at 37°C for 15 min; 40 µL of 0.6 mmol/L S-2765 solution was added, incubation was further conducted in a 37°C water bath for 15 min, then 40 µL of the stopping solution was added, and the absorbance at 405 nm was measured.

4.3 ATIII-IIa Activity test: 20 µL of ATIII solution and 20 µL of polysaccharide working solutions at different concentrations were added to each well (20 µL of Tris buffer was added to the blank well instead of the polysaccharide working solution), and incubation was conducted at 37°C for 10 min; 40 µL of IIa solution was added, and incubation was continued at 37°C for 10 min; 40 µL of 0.6 mmol/L S-2238 solution was added, incubation was conducted at 37°C for 15 min, then 40 µL of the stopping solution was added, and the absorbance at 405 nm was measured.

4.4 ATIII-Xa Activity test: 20 µL of ATIII solution and 20 µL of polysaccharide working solutions at different concentrations were added to each well (20 µL of Tris buffer was added to the blank well instead of the polysaccharide working solution), and incubation was conducted at 37°C for 10 min; 40 µL of Xa solution was added, and incubation was continued at 37°C for 10 min; 40 µL of 1 mmol/L S-2765 solution was added, and incubation was conducted at 37°C for 15 min, 40 µL of the stopping solution was then added, and the absorbance at 405 nm was measured.

### 5. Experimental Results:

The results of the IIa activity test, Xa activity test, ATIII-IIa activity test, and ATIII-Xa activity test are shown in Tables 18 to 21.

**Table 18 IIa Activity Test Results**

| Polysaccharide | Blank | 0.8 | 4 | 20 | 100 | 500 |
|---|---|---|---|---|---|---|
| concentration (µg/mL) | | | | | | |
| OD405 | 0.776 | 0.78 | 0.772 | 0.767 | 0.773 | 0.758 |

**Table 19 Xa Activity Test Results**

| Polysaccharide concentration (µg/mL) | Blank | 0.8 | 4 | 20 | 100 | 500 |
|---|---|---|---|---|---|---|
| OD405 | 1.81 | 1.84 | 1.879 | 1.862 | 1.796 | 1.870 |

**Table 20 ATIII-IIa Activity Test Results**

| Polysaccharide concentration (µg/mL) | Blank | 0.8 | 4 | 20 | 100 | 500 |
|---|---|---|---|---|---|---|
| OD405 | 0.707 | 0.695 | 0.705 | 0.681 | 0.649 | 0.629 |

**Table 21 ATIII-Xa Activity Test Results**

| Polysaccharide concentration (µg/mL) | Blank | 0.8 | 4 | 20 | 100 | 500 |
|---|---|---|---|---|---|---|
| OD405 | 0.806 | 0.793 | 0.769 | 0.753 | 0.744 | 0.51 |

The results in Tables 18 to 21 indicate that after HPS acted on IIa and Xa, the OD values of the administration groups at different concentrations and the blank control group show no significant decrease (Tables 18 to 19), indicating that HPS at an administration dose of 500 µg/mL displays no inhibitory activity against IIa and Xa, at an administration dose of 100 µg/mL shows no activity against ATIII-IIa and ATIII-Xa, and only exhibits weak inhibitory activity at an administration dose of 500 µg/mL (Tables 20 to 21), with inhibition rates of 11% and 36.7%, respectively.

### Example 10 Test of HPS in Inhibiting iFXase (intrinsic tenase) Activity

### 1. Test Objective

The primary pharmacological effect of HPS is as a non-heparin-like anticoagulant drug. Its action mechanism involves selectively inhibiting the terminal rate-limiting enzyme of the intrinsic coagulation pathway, "Intrinsic coagulation factor Xase (Intrinsictenase, FIXa-FVIIIa-PL-Ca²⁺ complex, iFXase)". In this example, the IC₅₀ value of HPS in inhibiting iFXase was determined.

As FVIII standard is readily available, the FVIII activity assay kit (FVIII:C) was used to determine the inhibitory effect of the test substance on iFXase activity.

### 2. Test Principle

In the presence of phospholipid (PLP) and calcium ions, VIII is activated by thrombin to form VIIIa. VIIIa, IXa, phospholipid, and calcium ions form the enzyme complex (iFXase), which subsequently activates Factor X. The generated Factor Xa hydrolyzes the chromogenic substrate SXa-11, releasing p-nitroaniline (pNA). pNA exhibits light absorption at 405 nm, and the amount of pNA is proportional to the absorbance at 405 nm. The activity of the enzyme complex is reflected by measuring the pNA content at 405 nm using a microplate reader. The logarithm of the enzyme complex concentration is proportional to A405. The amount of HPS that inhibits the activity of the enzyme complex formed by 1 U of FVIII is defined as 1 potency unit of the test substance. The test mechanism of the FVIII activity assay kit used in the present disclosure is shown in FIG. 8.

### 3. Instruments and Materials

The instruments required for the experiment are listed in Table 22, and the test drugs and kits required are listed in Table 23.

**Table 22 Instruments Required for the Experiment**

| Name | Model/specification | Manufacturer |
|---|---|---|
| Microplate reader | BioTeK SynergyH1 | Agilent |
| Pipette | 1000 µL | Eppendorf |
| Pipette | 200 µL | Eppendorf |
| Pipette | 20 µL | Eppendorf |
| Pipette | 10-100 µL eight-channel pipette | Eppendorf |
| Electronic multi-channel dispensing pipette | 1 µL-50 mL Multipette^{®}E3x | Eppendorf |
| Electronic balance | ME104T/02 | Mettler |

**Table 23 Test Drugs and Kits Required for the Experiment**

| Name | Batch No. | Trait | Specification | Source |
|---|---|---|---|---|
| HPS | 202401 | Freeze-dried powder | / | Harbin Hongdoushan Bio-pharm Co., Ltd., China |
| Human coagulation factor FVIII | 280016-202103 | Freeze-dried powder | 4.9 IU | China National Institutes for Food and Drug Control |
| FVIII Activity Assay Kit | 221402 | - | BIOPHENFVllI:C | BeiJing baiao innovation technology co., ltd., China |

### 4. Experimental Methods

### 4.1 Kit Solution Preparation:

2.5 mL of ultrapure water was separately added to R1 (FX), R2 (FIXa, FIIa, phospholipid, Tris-HCl, CaCl), and R3 (SXa-11), to obtain R1 solution, R2 solution, and R3 solution. R4 (Tris-BSA Buffer) was used for diluting the FVIII standard solution.

4.2 FVIII Working Solution Preparation: FVIII (4.9 IU/vial) was taken, and 1.225 mL of R4 reagent was pipetted to dissolve it, preparing a 4 IU/mL FVIII solution; 1,000 µL of the 4 IU/mL FVIII solution was taken, and 333 µL of R4 was added thereto to prepare a 3 IU/mL FVIII working solution.

### 4.3 Preparation of Polysaccharide Stock Solution and Working Solution

25 mg of the polysaccharide reference sample was accurately weighed and dissolved in 10 mL of ddH₂O to prepare Polysaccharide Stock Solution 1 (2.5 mg/mL); 100 µL of Polysaccharide Stock Solution 1 was pipetted and added to 9,900 µL of ddH₂O, and a resulting solution was diluted to Polysaccharide Stock Solution 2 (25 µg/mL); Polysaccharide Stock Solution 2 was pipetted and diluted with ddH₂O to prepare Polysaccharide Working Solution 1 (500 ng/mL), Working Solution 2 (300 ng/mL), Working Solution 3 (250 ng/mL), Working Solution 4 (180 ng/mL), Working Solution 5 (150 ng/mL), and Working Solution 6 (125 ng/mL).

4.4 Preparation of Reaction Stopping Solution: 1 g of citric acid was weighed and 50 mL of ddH₂O was added thereto to prepare a 2% citric acid stopping solution.

### 4.5 Polysaccharide Activity Determination

(1) 20 µL of ddH₂O was added to the blank well of a 96-well plate, and 20 µL of polysaccharide working solutions of different concentrations were sequentially added to the polysaccharide test wells of the 96-well plate;
(2) 20 µL of the 3 IU/mL FVIII factor working solution was sequentially added to the above test wells, followed by adding 20 µL of R2 solution to each well; after addition, the plate was incubated at 37°C for 15 min;
(3) 20 µL of R1 (FX) solution was added to each well and incubation was conducted at 37°C for 1 min; and
(4) 20 µL of R3 (Factor Xa chromogenic substrate SXa-11) solution was added to each well, incubation was conducted at 37°C for 15 min, and 30 µL of the stopping solution was then added thereto; the plate was placed in a microplate reader to measure the absorbance at 405 nm (A405).

### 5. Experimental Results:

The activity test results for polysaccharide at different unit concentrations are shown in Table 24. Based on the experimental data, the IC₅₀ value for polysaccharide in inhibition of iFXase activity was calculated using GraphPadPrism software as 207.5 ng/mL, with a confidence interval of 185.9-233 ng/mL.

**Table 24 Inhibition Effect of Polysaccharide on iFXase Activity and IC₅₀ Value**

| | | | | | | | |
|---|---|---|---|---|---|---|---|
| Concentration (µg/mL) | 0 | 0.125 | 0.15 | 0.18 | 0.25 | 0.3 | 0.5 |
| Absorbance | 0.989 | 0.824 | 0.672 | 0.557 | 0.395 | 0.238 | 0.16 |
| log(inhibitor) vs. normalized response--Variable slope | | | | | | | |
| Optimal fitted value | | | | | | | |
| LogIC₅₀ | | | | | -0.6830 | | |
| IC₅₀ | | | | | 0.2075 | | |
| 95%CI (likelihood profile) | | | | | | | |
| LogIC₅₀ | | | | | -0.7307 to -0.6327 | | |
| IC₅₀ | | | | | 0.1859 to 0.2330 | | |

The above results further clarify, at the mechanistic level, that the HPS of the present disclosure can selectively inhibit the activity of the rate-limiting enzyme iFXase in the intrinsic coagulation pathway, thereby targeting the intrinsic coagulation pathway.

The above are merely preferred embodiments of the present disclosure. It should be noted that a person of ordinary skill in the art may further make several improvements and modifications without departing from the principle of the present disclosure, but such improvements and modifications should be deemed as falling within the scope of the present disclosure.

## Claims

1. A Haina polysaccharide (HPS), wherein
the HPS is a fucosylated chondroitin sulfate polysaccharide;
the HPS has a weight-average molecular weight of 90,000 to 130,000, and is composed of monosaccharides comprising glucuronic acid, *N*-acetyl-*D*-galactosamine, and fucose, wherein a molar ratio of the glucuronic acid, the *N*-acetyl-*D*-galactosamine, and the fucose is in a range of 1 : 0.8-1.2 : 0.5-0.8; and
a sulfate group in the HPS has a mass percentage of 25% to 40%.

2. The HPS as claimed in claim 1, wherein the HPS has a structural unit shown in formula I: 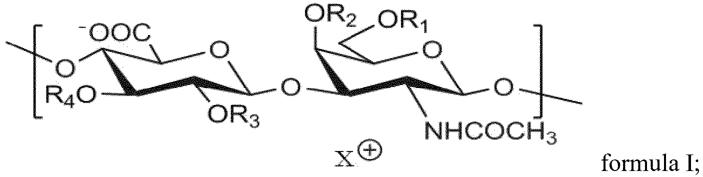
wherein in the formula I, R₁ is selected from the group consisting of H, SO₃⁻, and fucosyl; R₂ is selected from the group consisting of H and SO₃⁻; R₃ is selected from the group consisting of H and SO₃⁻; R₄ is selected from the group consisting of H and fucosyl; at least one of the R₁ and the R₄ is fucosyl; and X^{⊕} represents a cation; and
the fucosyl for the R₁ and the R₄ has a structure shown in formula I-1: 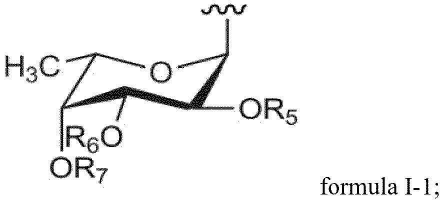
wherein in the formula I-1, R₅ is selected from the group consisting of H and SO₃⁻; R₆ is selected from the group consisting of H and SO₃⁻; and R₇ is selected from the group consisting of H and SO₃⁻.

3. The HPS as claimed in claim 2, wherein in a hydrogen nuclear magnetic resonance (¹H NMR) spectrum of the HPS, a signal peak for anomeric protons of a fucose moiety is present at a relative chemical shift of 5.70 ppm to 4.90 ppm; a signal peak for anomeric protons of a glucuronic acid moiety is present at a relative chemical shift of 4.80 ppm to 4.45 ppm; a signal peak for anomeric protons of an *N*-acetyl-*D*-galactosamine moiety is present at a relative chemical shift of 4.90 ppm to 4.50 ppm; a signal peak for acetyl protons of the N-acetyl-D-galactosamine moiety is present at a relative chemical shift of 2.40 ppm to 1.80 ppm; and a signal peak for methyl protons of the fucose moiety is present at a relative chemical shift of 1.60 ppm to 1.10 ppm;
in a carbon nuclear magnetic resonance (¹³C NMR) spectrum of the HPS, a signal peak for anomeric carbons of the glucuronic acid moiety is present at a relative chemical shift of 107.0 ppm to 105.0 ppm; a signal peak for anomeric carbons of the *N*-acetyl-*D*-galactosamine moiety is present at a relative chemical shift of 103.0 ppm to 101.0 ppm; a signal peak for anomeric carbons of the fucose moiety is present at relative chemical shifts of 104.0 ppm to 103.0 ppm and 101.0 ppm to 98.0 ppm; a signal peak for 2-position carbon of the *N*-acetyl-*D*-galactosamine moiety is present at a relative chemical shift of 56.0 ppm to 51.0 ppm; a signal peak for acetyl carbons of the *N*-acetyl-*D*-galactosamine moiety is present at a relative chemical shift of 27.0 ppm to 24.0 ppm; and a signal peak for methyl carbon of the fucose moiety is present at a relative chemical shift of 20.0 ppm to 16.0 ppm; and
during measurement of the ¹H NMR spectrum and the ¹³C NMR spectrum of the HPS, deuterium oxide is used as a solvent, and sodium 3-(trimethylsilyl)propionate-2,2,3,3-d4 is used as an internal standard.

4. The HPS as claimed in claim 2, wherein the cation is selected from the group consisting of hydrogen ion, an alkali metal ion, an alkaline earth metal ion, an ammonium ion, and an iron ion.

5. The HPS as claimed in claim 2, wherein the cation is sodium ion; and the sodium ion in the HPS has a mass percentage of 5% to 15%.

6. The HPS as claimed in claim 1, wherein the HPS has a polydispersity index less than or equal to 2.

7. A preparation method of the HPS as claimed in any one of claims 1 to 6, comprising the steps of:
(1) subjecting a body wall powder of a sea cucumber to enzymolysis and acid hydrolysis in sequence to obtain an acid hydrolysate supernatant, wherein an enzyme used in the enzymolysis is a protease;
(2) adjusting a pH value of the acid hydrolysate supernatant to 6.5 to 7.5, conducting alcohol precipitation, and subjecting an obtained alcohol precipitation solid to washing, centrifugation, and freeze-drying in sequence to obtain a crude polysaccharide product; and
(3) subjecting the crude polysaccharide product to column chromatography purification, collecting a fraction having a weight-average molecular weight of 90,000 to 130,000, and subjecting the fraction to alcohol precipitation to obtain the HPS.

8. The preparation method as claimed in claim 7, wherein the sea cucumber comprises one or more selected from the group consisting of *Holothuria leucospilota, Holothuria scabra, Stichopus japonicus, Holothuria atra, and Holothuria nobilis.*

9. Use of the HPS as claimed in any one of claims 1 to 6 or the HPS prepared by the preparation method as claimed in any one of claims 7 to 8 in manufacture of an anticoagulant drug and/or an antithrombotic drug or an inhibitor targeting intrinsic tenase (iFXase).

10. An inhibitor targeting iFXase, wherein an active ingredient of the inhibitor targeting iFXase comprises the HPS as claimed in any one of claims 1 to 6 or the HPS prepared by the preparation method as claimed in any one of claims 7 to 8.

11. An anticoagulant drug and/or an antithrombotic drug targeting an intrinsic coagulation pathway, comprising: an active ingredient and a pharmaceutically acceptable auxiliary material, wherein the active ingredient is the HPS as claimed in any one of claims 1 to 6 or the HPS prepared by the preparation method as claimed in any one of claims 7 to 8.

12. The anticoagulant drug and/or the antithrombotic drug targeting the intrinsic coagulation pathway as claimed in claim 11, wherein a dosage form of the anticoagulant drug and/or the antithrombotic drug targeting the intrinsic coagulation pathway is selected from the group consisting of a tablet, a powder, a capsule, a granule, and a liquid preparation.

13. A method for treating a vascular disease, comprising: administering the HPS as claimed in any one of claims 1 to 6, the inhibitor targeting coagulation factor Xase as claimed in claim 10, or the anticoagulant drug and/or the antithrombotic drug targeting the intrinsic coagulation pathway as claimed in any one of claims 11 to 12 for treatment.

14. The method as claimed in claim 13, wherein an administration dose of the HPS is in a range of 10 mg/day/person to 30 mg/day/person; and
in terms of the HPS, the inhibitor targeting coagulation factor Xase or the anticoagulant drug and/or the antithrombotic drug targeting the intrinsic coagulation pathway has an administration dose of 10 mg/day/person to 30 mg/day/person.

15. The method as claimed in claim 13, wherein the vascular disease comprises a cardiovascular and cerebrovascular disease.

16. The method as claimed in claim 13, wherein the vascular disease comprises a thrombotic disease.

17. The method as claimed in claim 16, wherein the thrombotic disease comprises ischemic stroke.
